# EUROPEAN PATENT APPLICATION

(11) **EP 4 389 864 A1**
(43) Date of publication of application: **26.06.2024**
(21) Application number: 22214976.7
(22) Date of filing: 20.12.2022
(51) Int. Cl.: C11D 3/386

(54) **CUTINASES**

(71) Applicant: BASF SE, 67056 Ludwigshafen am Rhein (DE)
(72) Inventor: Kline, Katie, San Diego, CA 19121 (US); Hoang, Cindy, San Diego, CA 19121 (US); Caglic, Dejan, San Diego, CA 19121 (US); Liszka, Michael, San Diego, CA 19121 (US); Nielsen, Jesper, San Diego, CA 19121 (US)
(74) Representative: BASF IP Association

(57) **Abstract**

In the present invention cutinase enzymes are provided. Additionally, compositions comprising cutinase enzymes and methods of making and using cutinase enzymes are provided.

## Description

### Field of the invention

In the present invention cutinase enzymes are provided. Additionally, compositions comprising the cutinase enzymes and methods of making and using the cutinase enzymes are provided.

### Background of the invention

Enzymes are biodegradable and therefore are increasingly used in various applications as sustainable alternatives to petrochemistry. Enzymes can be catalytically active even at lower temperatures, which can result in an overall reduction of energy consumption. In particular, in the detergent industry enzymes are incorporated in detergent compositions to improve cleaning efficiency and to reduce energy consumption in the washing step.

Cutinases are enzymes capable of e. g. hydrolyzing cutin, waxy polymers that are the main components of the plant cuticle, and other fatty substrates. Thus, cutinases have been suggested to be used as potential detergent enzymes. Nevertheless, up to now, cutinases are rarely used in the detergent industry as they are rarely stable at different temperatures and/or within the denaturing conditions of the detergents and the wash liquor. Thus, the need exists for new cutinase enzymes, which meet these requirements.

There is an additional need to find new detergent additives that facilitate removal of fatty stains, in particular sebum stains.

### Brief summary of the invention

Thus, the present invention is directed to new polypeptides having cutinase activity and compositions comprising these cutinases, wherein the polypeptide having cutinase activity comprises an amino acid sequence that is at least 75% identical to SEQ ID NO: 2, at least 80% identical to SEQ ID NO: 4, or at least 84% identical to SEQ ID NO: 6. Additionally, methods for removing plant-based stains and/or fatty stains, preferably fatty stains, preferably sebum stains, using a composition comprising such cutinases are provided.

### Detailed description of the invention

The present invention may be understood more readily by reference to the following detailed description of the embodiments of the invention and the examples included herein.

Although the present invention will be described with respect to particular embodiments, this description is not to be construed in a limiting sense.

### Definitions

Unless otherwise noted, the terms used herein are to be understood according to conventional usage by those of ordinary skill in the relevant art.

Before describing exemplary embodiments of the present invention in detail, definitions important for understanding the present invention are provided. Unless stated otherwise or apparent from the nature of the definition, the definitions apply to all compounds, methods and uses described herein.

As used in this specification and in the appended claims, the singular forms of "a" and "an" also include the respective plurals and vice versa unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ± 20%, preferably ± 15%, more preferably ± 10%, and even more preferably ± 5%. Furthermore, the terms "first", "second", "third" or "(a)", "(b)", "(c)", "(d)" etc. in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay, there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

Throughout this application, various publications are referenced. The disclosures of all these publications and the references cited within those publications in their entireties are hereby incorporated by reference into this application to describe the state of the art to which this invention pertains more fully.

It is to be understood that the term "comprising" is not limiting. For the purposes of the present invention the term "consisting of" is considered to be a preferred embodiment of the term "comprising". If hereinafter a group is defined to comprise at least a certain number of members, this is meant to also encompass a group which consists of these members only.

In describing the polypeptides of the present invention, the abbreviations for single amino acids used are according to the accepted IUPAC single letter or three letter amino acid abbreviation. "Amino acid alteration" as used herein refers to amino acid substitution, deletion, or insertion. "Substitutions" are described by providing the original amino acid followed by the number of the position within the amino acid sequence followed by the amino acid, which substitutes the original amino acid. For example, the substitution of histidine at position 120 with alanine is designated as "His120Ala" or "H120A". Substitutions can also be described by merely naming the resulting amino acid without specifying the initial amino acid at this position, e.g., "X120A" or "120A" or "Xaa120Ala" or "120Ala".

"Deletions" are described by providing the original amino acid followed by the number of the position within the amino acid sequence followed by *. Accordingly, the deletion of glycine at position 150 is designated as "Gly150*" or "G150*". Alternatively, deletions are indicated by, e.g., "deletion of D183 and G184".

"Insertions" are described by providing the original amino acid followed by the number of the position within the amino acid sequence followed by the original amino acid and the additional amino acid. For example, an insertion at position 180 of lysine next to glycine is designated as "Gly180GlyLys" or "G180GK". When more than one amino acid residue is inserted, such as, e.g., a Lys and an Ala after Gly180 this may be indicated as: "Gly180GlyLysAla" or "G195GKA". In cases where a substitution and an insertion occur at the same position, this may be indicated as "S99A+S99SD" or in short "S99AD". Sequences comprising multiple alterations are separated by "+", e.g., "Arg170Tyr+Gly195Glu", "R170Y+G 195E" or "X170Y+X195E" representing a substitution of arginine and glycine at positions 170 and 195 with tyrosine and glutamic acid, respectively. Alternatively, multiple alterations may be separated by space or a comma, e.g., "R170Y G195E" or "R170Y, G195E" respectively. Where different alternative alterations can be introduced at a position, the different alterations are separated by a comma, e.g., "Arg170Tyr, Glu" and "R170T, E", respectively, represents a substitution of arginine at position 170 with tyrosine or glutamic acid. Alternative substitutions at a particular position can also be indicated as "X120A,G,H", "120A,G,H", "X120A/G/H", or "120A/G/H". Alternatively, different alterations or optional substitutions may be indicated in brackets, e.g., "Arg170 [Tyr, Gly]" or "Arg170 {Tyr, Gly}" or in short "R170 [Y, G]" or "R170 {Y, G}".

The term "native" (or naturally or wildtype or endogenous) cell or organism or polynucleotide or polypeptide refers to the cell or organism or polynucleotide or polypeptide as found in nature (i.e., without any human intervention).

The term "isolated" molecule, e. g. polypeptide or polynucleotide, is defined herein as a molecule, which has been separated from its natural environment.

The term "heterologous polypeptide" (or exogenous or foreign polypeptide) is defined herein as a polypeptide, which is naturally not expressed by a host cell. The term "heterologous nucleotide" (or exogenous or foreign polynucleotide) is defined herein as a polynucleotide, which is naturally not contained in a host cell.

For the purpose of the invention, "recombinant" (or non-native or non-naturally) with regards to a cell or an organism means that the cell or organism contains a polynucleotide, which is introduced using gene technology. Recombinant with regards to a polynucleotide or a polypeptide means that the polynucleotide or polypeptide has been newly combined or rearranged in terms of its genetic environment by using recombinant DNA techniques. Thus, recombinant polynucleotides or polypeptides include a polypeptide or polynucleotide native to the host cell, whose expression is quantitatively altered or whose expression is directed from a genomic location different from the native host cell as a result of manipulation of the DNA of the host cell by recombinant DNA techniques, e.g., a stronger promoter. Recombinant polynucleotides or polypeptides also can be heterologous, meaning they can be foreign sequences, but they can also originate from the same organism that they are introduced to.

With respect to the relation between two or more polynucleotides or the relation between two or more polypeptides, the term "recombinant" is used to characterize that the two or more polynucleotides or two or more polypeptides are naturally not occurring in the specific combination with each other.

"Modified recombinant" polynucleotides or polypeptides means recombinant polynucleotides or recombinant polypeptides that have been modified by introducing alterations, e.g., deletions, substitutions, and/or insertions, using recombinant DNA techniques to alter the native polypeptide or native polynucleotide.

A "synthetic" compound is obtained by in vitro chemical and/or enzymatic synthesis.

Sequence identity usually is provided as "% sequence identity" or "% identity". For calculation of sequence identities, in a first step a sequence alignment is produced. According to this invention, a pairwise global alignment is produced, meaning that two sequences are aligned over their complete length. The alignment is usually produced by using a mathematical approach called alignment algorithm.

According to the invention, the alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used for the purposes of the current invention, using the programs default parameter (polynucleotides: gap open=10.0, gap extend=0.5 and matrix=EDNAFULL; polypeptides: gap open=10.0, gap extend=0.5 and matrix=EBLOSUM62). After aligning the two sequences, an identity value is determined from the produced alignment in a second step. For this purpose, the %-identity is calculated by dividing the number of identical residues by the length of the alignment region, which is showing the respective sequence of the present invention over its complete length multiplied with 100: %-identity = (identical residues / length of the alignment region, which is showing the respective sequence of the present invention over its complete length) *100.

For calculating the percent identity of two polynucleotides the same applies as outlined above with some specifications. For polynucleotides encoding for a protein the pairwise alignment shall be made over the complete length of the coding region of the sequence of this invention from start to stop codon excluding introns. Introns present in the other sequence, to which the sequence of this invention is compared to shall also be removed for the pairwise alignment. After aligning the two polynucleotide sequences, an identity value is determined in a second step from the alignment produced. Percent identity is calculated by: %-identity = (identical residues / length of the alignment region, which is showing the sequence of the invention from start to stop codon excluding introns over its complete length) *100.

Herein, the exchange of one amino acid with a similar amino acid may be called "conservative mutation". Similar amino acids according to the invention are defined as follows:
Amino acid A is similar to amino acids S
Amino acid D is similar to amino acids E; N
Amino acid E is similar to amino acids D; K; Q
Amino acid F is similar to amino acids W; Y
Amino acid H is similar to amino acids N; Y
Amino acid I is similar to amino acids L; M; V
Amino acid K is similar to amino acids E; Q; R
Amino acid L is similar to amino acids I; M; V
Amino acid M is similar to amino acids I; L; V
Amino acid N is similar to amino acids D; H; S
Amino acid Q is similar to amino acids E; K; R
Amino acid R is similar to amino acids K; Q
Amino acid S is similar to amino acids A; N; T
Amino acid T is similar to amino acids S
Amino acid V is similar to amino acids I; L; M
Amino acid W is similar to amino acids F; Y
Amino acid Y is similar to amino acids F; H; W

A "fragment" or "subsequence" as used herein is a portion of a polynucleotide or an amino acid sequence. The term "functional fragment" refers to any nucleic acid or amino acid sequence, which merely comprises a part of the full-length amino acid sequence, but still has the same or similar activity and/or function. Preferably, the functional fragment is at least 75%, at least 76%, at least 77%, at least 78%, at least 79%, at least 80% identical, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.5 %, at least 99%, or at least 99.5% identical to the original full length amino acid sequence. The functional fragment comprises consecutive nucleotides or amino acids identical to the respective part of the original nucleic acid or original amino acid sequence.

"Genetic construct" or "expression cassette" as used herein, is a nucleic acid molecule composed of at least one sequence of interest to be expressed, operably linked to one or more control sequences (at least to a promoter) as described herein.

The term "vector" as used herein comprises any kind of construct suitable to carry polynucleotide sequences for transfer to a cell, or for stable or transient expression within a given cell. The term "vector" as used herein encompasses any kind of cloning vehicles, such as but not limited to plasmids, phagemids, viral vectors (e.g., phages), bacteriophage, baculoviruses, cosmids, fosmids, artificial chromosomes, and any other vectors specific for specific hosts of interest. Foreign polynucleotide sequences usually comprise a coding sequence, which may be referred to herein as "gene of interest". The gene of interest may comprise introns and exons, depending on the kind of origin or destination of host cell.

The term "introduction of a polynucleotide" or "transformation of a polynucleotide" as referred to herein encompasses the transfer of a polynucleotide outside a host cell into a host cell, irrespective of the method used for transfer. That is, the term "transformation of a polynucleotide" as used herein is independent from vector, shuttle system, or host cell. Furthermore, it does not only relate to the polynucleotide transfer method of transformation (cf., for example, Sambrook, J. etal. (1989) Molecular Cloning: A Laboratory Manual, 2nd Ed., Cold Spring Harbor Laboratory Press, Cold Spring Harbor, NY), but it encompasses any further kind of polynucleotide transfer methods such as, but not limited to, transduction or transfection.

A polynucleotide encoding a polypeptide may be "expressed". The term "expression" or "gene expression" means the transcription of a gene or genes or genetic construct into structural RNA (e.g., rRNA, tRNA) or mRNA with or without subsequent translation of the latter into a protein. The process includes transcription of DNA and processing of the resulting mRNA product.

The term "purification" or "purifying" refers to a process in which at least one component, e.g., a protein of interest, is separated from at least another component, e.g., a particulate matter of a fermentation broth, and transferred into a different compartment or phase, wherein the different compartments or phases do not necessarily need to be separated by a physical barrier. Examples of such different compartments are two compartments separated by a filtration membrane or cloth, i.e., filtrate and retentate; another example of such different phases are pellet and supernatant or cake and filtrate, respectively. The resulting solution after purifying the enzyme of interest from the fermentation broth is called herein "purified enzyme solution".

"Protein formulation" (or "protein preparation"), means any non-complex formulation comprising a small number of ingredients, wherein the ingredients serve the purpose of stabilizing the proteins comprised in the protein formulation and/or the stabilization of the protein formulation itself. Preferably, the non-complex protein formulation comprises the protein in higher concentrations than the complex composition, e.g., than a detergent composition. Thus, preferably the non-complex protein formulation is a concentrated protein formulation. Preferably, non-complex protein formulations comprise 0.1 to 40% enzyme by weight, whereas complex formulations, like detergent compositions, comprise 0.0002 to 0.09% enzyme by weight, all relative to the total weight of the enzyme formulation.

"Enzyme properties" include, but are not limited to catalytic activity, substrate/cofactor specificity, product specificity, stability in the course of time, thermostability, pH stability, and chemical stability. "Enzymatic activity" or "catalytic activity" means the catalytic effect exerted by an enzyme, expressed as units per milligram of enzyme (specific activity) or molecules of substrate transformed per minute per molecule of enzyme (molecular activity). Enzymatic activity can be specified by the enzymes actual function, e.g., proteases exerting proteolytic activity by catalyzing hydrolytic cleavage of peptide bonds, lipases exerting lipolytic activity by hydrolytic cleavage of ester bonds, amylases activity involves hydrolysis of glycosidic linkages in polysaccharides, cutinase activity involves hydrolysis of ester bonds in omega hydroxy acids and their derivatives etc.

The term "enzyme stability" according to the current invention relates to the retention of enzymatic activity as a function of time during storage or operation. Retention of enzymatic activity as a function of time during storage is called "storage stability" and is a preferred embodiment of enzyme stability within the context of the invention.

To determine and quantify changes in catalytic activity of enzymes stored or used under certain conditions over time, the "initial enzymatic activity" is measured under defined conditions at time zero (100%) and at a certain point later in time (x%). By comparing these values, a potential loss of enzymatic activity can be determined. The extent of loss of enzymatic activity determines an enzyme's stability or non-stability.

"Enzyme inhibitors" as used herein are compounds that slow down or halt enzymatic activity. Enzyme inhibitors frequently also stabilize the enzyme in its three-dimensional structure. Hence, enzyme inhibitors usually also act as "enzyme stabilizers".

"pH stability" refers to the ability of an enzyme to exert enzymatic activity after exposure to a certain pH value.

The terms "thermal stability", "thermostability" or "temperature-dependent activity" refer to the ability of an enzyme to exert catalytic activity or wash performance after exposure to elevated temperatures, preferably, at a temperature of 37-45 °C for 28 days, more preferably 56 days, preferably in a detergent composition (preferably, in model ES1-C detergent).

The terms "detergent stability" or "stability under storage in a detergent composition" refer to the ability of an enzyme to exert catalytic activity or wash performance after storage in a detergent composition (preferably, in model ES1-C detergent), preferably, at a temperature of 37 °C or 45 °C for 28 days, more preferably 56 days.

As used herein, "wash performance" (also called "cleaning performance" herein) of an enzyme refers to the contribution of the enzyme to the cleaning performance of a detergent composition, i.e. the cleaning performance added to the detergent composition by the performance of the enzyme. The term "wash performance" is used similarly herein for laundry and hard surface cleaning. Wash performance is compared under relevant washing conditions. The term "relevant washing conditions" is used herein to indicate the conditions, particularly washing temperature, time, washing mechanics, concentration of detergent components in the wash liquor (i.e., suds concentration), type of detergent and water hardness, used in households in a detergent market segment. The term "improved wash performance" is used to indicate that a better cleaning performance is obtained under relevant washing conditions, or that less enzyme, on weight basis, is needed to obtain the same end result relative to the corresponding control conditions.

As used herein, the term "specific performance" refers to the cleaning and removal of specific stains or soils per unit of active enzyme. In some embodiments, the specific performance is determined using stains or soils such as egg, egg yolk, milk, grass, spinach, minced meat, blood, chocolate sauce, baby food, sebum, etc.

"Detergent composition" or "detergent" means compositions designated for cleaning soiled material. Detergent compositions according to the invention include detergent compositions for different applications such as laundry and hard surface cleaning. The term "detergent component" is defined herein to mean a type of chemical, which can be used in detergent compositions. A typical detergent component is a surfactant. "Surfactant" (synonymously used herein with "surface active agent") means an organic chemical that, when added to a liquid, changes the properties of that liquid at an interface. According to its ionic charge, a surfactant is called non-ionic, anionic, cationic, or amphoteric. The term "effective amount of a detergent component" includes amounts of certain components to provide effective stain removal and/or effective cleaning conditions (e. g. pH, temperature, water hardness, quantity of foaming), amounts of certain components to effectively provide optical benefits (e. g. optical brightening, dye transfer inhibition, color care), and amounts of certain components to effectively aid the processing (maintain physical characteristics during processing, storage and use; e.g. rheology modifiers, hydrotropes, desiccants).

The term "laundry" or "laundering" relates to both household cleaning and industrial cleaning and means the process of treating textiles and/or fabrics with a solution containing a detergent composition of the present invention. The cleaning or washing process may be carried out by using technical devices such as a household or an industrial washing machine. Alternatively, the cleaning or washing process may be done by hand.

The term "textile" means any textile material including yarns (thread made of natural or synthetic fibers used for knitting or weaving), yarn intermediates, fibers, non-woven materials, natural materials, synthetic materials, as well as fabrics made of these materials such as garments, cloths and other articles. The terms "fabric" (a textile made by weaving, knitting or felting fibers) or "garment" (any article of clothing made of textile) as used herein, mean to include the broader term textile as well.

The term "fibers" includes natural fibers, synthetic fibers, and mixtures thereof. Examples of natural fibers are of plant (such as flax, jute and cotton) or animal origin, the latter comprising proteins like collagen, keratin and fibroin (e. g. silk, sheep wool, angora, mohair, cashmere). Examples for fibers of synthetic origin are polyurethane fibers such as Spandex^{®} or Lycra^{®}, polyester fibers, polyolefins such as elastofin, or polyamide fibers such as nylon. Fibers may be single fibers or parts of textiles such as knitwear, woven or non-woven fabrics.

The term "hard surface cleaning" relates to both household hard surface cleaning and industrial hard surface cleaning and means the process of treating hard surfaces with a solution containing a detergent composition of the present invention. Hard surfaces may include any hard surfaces in a household or industry, such as floors, furnishing, walls, sanitary ceramics, glass, metallic surfaces including medical devices, cutlery, and dishes. A particular form of hard surface cleaning is dishwashing, including manual dish washing (MDW) or automatic dishwashing (ADW).

The term "dish wash" refers to all forms of washing dishes, e. g. by hand or automatic dish washing. Washing dishes includes, but is not limited to, the cleaning of all forms of crockery such as plates, cups, glasses, bowls, all forms of cutlery such as spoons, knives, forks and serving utensils as well as ceramics, plastics such as melamine, metals, china, glass, and acrylics.

Cleaning performance is evaluated under relevant cleaning conditions. The term "relevant cleaning conditions" herein refers to the conditions, particularly cleaning temperature, time, cleaning mechanics, suds concentration, type of detergent and water hardness used in laundry machines, automatic dish washers or in manual cleaning processes.

The term "medical device cleaning" refers to the cleaning step in reprocessing reusable medical devices. Medical device cleaning methods can be divided into two categories, manual and mechanical/automated cleaning methods. Manual cleaning is used when mechanical units are not available or medical devices to be cleaned are too fragile or difficult to clean with a mechanical unit. Mechanical/automated cleaning methods remove soiling and microorganisms through an automated cleaning and rinsing process, this includes ultrasonic cleaning and washing.

In the field of detergency, usually the term "stains" is used with reference to laundry, e.g., cleaning for textiles, fabric, or fibers, whereas the term "soils" is usually used with reference to hard surface cleaning, e.g., cleaning of dishes and cutlery. However, herein the terms "stain" and "soil" are used interchangeably.

"Fatty stains" are fat containing stains. Fat means any ester of fatty acids. Therefore, fatty stains refer but are not limited to oily stains, solid fat stains, sebum stains, and body soil stains. Sebum stains preferably contain lipids, preferably comprising at least one of triglycerides, wax esters, squalene and/or cholesterol. More preferably, sebum stains comprise a mixture of lipids, preferably one or more of triglycerides, wax esters, squalene and/or cholesterol, preferably a mixture of triglycerides, wax esters, squalene and cholesterol.

A "sequestering builder" as used herein is different from a precipitating builder in that no significant amount of precipitate is formed when the sequestering builder is used in an amount sufficient to combine with all the calcium ions in an aqueous solution with 7 °dH hardness (German hardness) starting with a neutral pH. A "strong builder" is classified as high efficiency chelator that strongly binds divalent cations such as Ca²⁺ with a logarithmic stability constant (Log K_{Ca}) of the cation/chelator complex of above 4, particular above 5, above 6 or above 7. The stability constants are determined at an ionic strength of 0.1 M and at a temperature of 25 °C. A "strong sequestering builder" combines both above-mentioned properties.

A composition "essentially devoid" of a compound shall mean herein that the respective compound is not added to the composition on purpose, preferably that at most non-effective amounts are present, most preferably 0% of the compound are contained in the composition.

### Detailed description

In the present invention cutinase enzymes are provided. More specifically, compositions comprising the cutinase enzymes and methods of making and using the cutinase enzymes are provided. Preferably, the cutinase of the present invention is a purified, isolated, synthetic, and/or recombinant cutinase. Preferably, the cutinase of the present invention is a purified and recombinant cutinase.

Cutinases according to the invention have "cutinase activity". "Cutinase activity" describes the capability for hydrolyzing ester bonds. Initially, cutinase activity has been associated with the hydrolysis of ester bonds of the plant polymer cutin. However, cutinases have been shown to also catalyze the hydrolysis of other polymers, triacylglycerols, and low-molecular-weight soluble esters. Cutinases have also been shown to be able to catalyze the hydrolysis of PET (polyethylene terephthalate)). Cutinases are hydrolases that are classified in the EC class 3.1.1.74.

Cutinase activity may be determined by assays for measurement of cutinase activity, which are known to those skilled in the art. For instance, cutinase activity can be determined using p-nitrophenyl butyrate or octanoic acid 4-nitrophenyl ester as a substrate. Reaction rates of enzymatic hydrolysis can be determined by monitoring the release of p-nitrophenol spectrophotometrically at 405 nm in a spectrophotometer.

The cutinase of the present invention is a cutinase that comprises an amino acid sequence that is at least 75% identical to SEQ ID NO: 2, at least 80% identical to SEQ ID NO: 4, or at least 84% identical to SEQ ID NO: 6.

In one embodiment, the cutinase comprises an amino acid sequence that is with increasing preference at least 76%, at least 78%, at least 80%, at least 82%, at least 85%, at least 87%, at least 90%, at least 92%, at least 95%, at least 96%, at least 97%, at least 98% at least 99%, or 100% identical to SEQ ID NO: 2.

In another embodiment, the cutinase comprises an amino acid sequence that is with increasing preference at least 82%, at least 85%, at least 87%, at least 90%, at least 92%, at least 95%, at least 96%, at least 97%, at least 98% at least 99%, or 100% identical to SEQ ID NO: 4.

In another embodiment, the cutinase comprises an amino acid sequence that is with increasing preference at least 85%, at least 87%, at least 90%, at least 92%, at least 95%, at least 96%, at least 97%, at least 98% at least 99%, or 100% identical to SEQ ID NO: 6.

Preferably, the cutinase comprises an amino acid sequence that is with increasing preference at least 90%, at least 91%, at least 92%, at least 93%, at least 94%, at least 95%, at least 96%, at least 97%, at least 98%, at least 99% or 100% identical to SEQ ID NO: 2, 4 or 6.

More preferably, the cutinase comprises an amino acid sequence that is with increasing preference at least 90%, at least 91%, at least 92%, at least 93%, at least 94%, at least 95%, at least 96%, at least 97%, at least 98%, at least 99% or 100% identical to SEQ ID NO: 2 or 6.

Most preferably, the cutinase comprises an amino acid sequence that is with increasing preference at least 90%, at least 91%, at least 92%, at least 93%, at least 94%, at least 95%, at least 96%, at least 97%, at least 98%, at least 99% or 100% identical to SEQ ID NO: 2.

In one embodiment, the cutinase comprises or consists of an amino acid sequence that is 100% identical to SEQ ID NO: 2, 4 or 6, preferably SEQ ID NO: 2 or SEQ ID NO: 6, more preferably SEQ ID NO: 2.

In one embodiment, the cutinase comprises or consists of an amino acid sequence that is 100% identical to SEQ ID NO: 2, 4 or 6, preferably SEQ ID NO: 2, but comprising 1-20, preferably, 1-15, more preferably 1-10, or even more preferably 1-5 amino acid substitutions, preferably conservative amino acid substitutions.

Ways of introducing amino acid alterations, e.g., substitutions, preferably conservative substitutions, into protein sequence are well known in the art. Substitutions can be introduced by using any mutagenesis procedure known in the art, such as site-directed mutagenesis, synthetic gene construction, semi-synthetic gene construction, random mutagenesis, shuffling, etc., followed by a relevant screening procedure. See, *e.g.,* Scherer and Davis, 1979, Proc. Natl. Acad. Sci. USA 76: 4949-4955; and Barton et al., 1990, Nucleic Acids Res. 18: 7349-4966, U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16, Tian et al, 2004, Nature 432: 1050-1054; Reidhaar-Olson and Sauer, 1988, Science 241: 53-57; Bowie and Sauer, 1989, Proc. Natl. Acad. Sci. USA 86: 2152-2156; WO 95/17413; or WO 95/22625, Lowman et al., 1991, Biochemistry 30: 10832-10837; U.S. Patent No. 5,223,409; WO 92/06204, Derbyshire et al., 1986, Gene 46: 145; Ner et al., 1988, DNA 7: 127.

### Nucleic acid construct

The present invention also refers to a polynucleotide encoding the cutinase of the present invention or the complement thereof. Preferably, the polynucleotide is a codon-optimized polynucleotide for improving expression in a specific host cell, preferably a Bacillus cell.

The present invention thus also refers to a polynucleotide, preferably an isolated, a synthetic, and/or a recombinant polynucleotide comprising:
(a) a polynucleotide having with increasing preference at least 60%, at least 65%, at least 70%, at least 75%, at least 80%, at least 85%, at least 90%, at least 91 %, at least 92%, at least 93%, at least 94%, at least 95%, at least 96%, at least 97%, at least 98%, at least 99% or 100% identity to SEQ ID NO: 1, SEQ ID NO: 3, or SEQ ID NO: 5, wherein the polynucleotide encodes a polypeptide having cutinase activity;
(b) a polynucleotide encoding a polypeptide having with increasing preference at least 85%, at least 90%, at least 91 %, at least 92%, at least 93%, at least 94%, at least 95%, at least 96%, at least 97%, at least 98%, at least 99%, or 100% identity to SEQ ID NO: 2, SEQ ID NO: 4, or SEQ ID NO: 6, wherein the polypeptide has cutinase activity;
(c) a polynucleotide that hybridizes under high stringency conditions, preferably under very high stringency conditions, with the complement of
   (i) a coding sequence of SEQ ID NO: 2, SEQ ID NO: 4, or SEQ ID NO: 6; or
   (ii) a polynucleotide shown in SEQ ID NO: 1, SEQ ID NO: 3, or SEQ ID NO: 5;
(d) a fragment of (a), (b), or (c), wherein the fragment encodes a polypeptide having cutinase activity; or
(e) a polynucleotide sequence fully complementary to any of (a) to (d).
(f) a polynucleotide that differs from any of the nucleic acid sequences described in (a) to (e) merely by the degeneracy of the genetic code.

In a further embodiment, the invention also relates to polypeptides having cutinase activity which are encoded by a polynucleotide described herein, preferably isolated, synthetic or recombinant polypeptides having cutinase activity, which are encoded by a polynucleotide described herein. Preferably, the polypeptide having cutinase activity is encoded by a polynucleotide having with increasing preference at least at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 1, SEQ ID NO: 3, or SEQ ID NO: 5.

In another embodiment, the polypeptide having cutinase activity is encoded by a polynucleotide that hybridizes under high stringency conditions, preferably under very high stringency conditions, with the full-length complement of a polynucleotide having with increasing preference at least at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to SEQ ID NO: 1, SEQ ID NO: 3, or SEQ ID NO: 5..

The present invention also refers to a nucleic acid construct, preferably an expression cassette, comprising the polynucleotide as described herein.

Typically, the expression cassette comprises three elements: a promoter sequence, an open reading frame, and a 3' untranslated region that, in eukaryotes, usually contains a polyadenylation site. Additional regulatory elements may include transcriptional as well as translational enhancers. An intron sequence may also be added to the 5' untranslated region (UTR) or to the coding sequence to increase the amount of the mature message that accumulates in the cytosol. The expression cassette may be part of a vector or may be integrated into the genome of a host cell and replicated together with the genome of its host cell. The expression cassette usually is capable of increasing or decreasing expression.

The present invention also refers to an expression vector comprising the polynucleotide or the nucleic acid construct as described herein. The expression vector can be a low copy number vector or high copy number vector.

A vector as used herein may provide segments for transcription and translation of a polynucleotide upon transformation into a host cell or host cell organelles. Such additional segments may include regulatory nucleotide sequences, one or more origins of replication that is required for its maintenance and/or replication in a specific cell type, one or more selectable markers, a polyadenylation signal, a suitable site for the insertion of foreign coding sequences such as a multiple cloning site etc. One example is vector being required to be maintained in a bacterial cell as an episomal genetic element (e.g., plasmid or cosmid molecule). Non-limiting examples of suitable origins of replication include the f1-ori and colE1.

A vector may replicate without integrating into the genome of a host cell, e.g., as a plasmid in a bacterial host cell, or it may integrate part or all of its DNA into the genome of the host cell and thus lead to replication and expression of its DNA.

The polynucleotide encoding the cutinase may be introduced into a vector by means of standard recombinant DNA techniques. Once introduced into the vector, the polynucleotide comprising a coding sequence may be suitable to be introduced (transformed, transduced, transfected, etc.) into a host cell or host cell organelles. A cloning vector suitable for expression of the polynucleotide sequence in the host cell or host cell organelles may be chosen.

### Host cell

The present invention also refers to a host cell comprising a polynucleotide encoding the cutinases as described herein, the nucleic acid construct as described herein, or the expression vector as described herein. In one embodiment of the invention, a vector is used for transformation of a host cell.

The polynucleotides encoding the cutinases as described herein may be stably or transiently introduced into a host cell and may be maintained non-integrated, for example, as a plasmid. Usually, stable transformation is due to integration of a nucleic acid comprising a foreign coding sequence into a chromosome or as an episome (separate piece of nuclear DNA). Usually, during transient transformation the nucleic acid comprising the foreign nucleic acid sequence is not integrated into a chromosome or as an episome.

The introduction of nucleic acid into a host cell may, for instance, but not limited thereto, be effected by protoplast transformation (see, e.g., Chang and Cohen, 1979, Molecular General Genetics 168: 111-115), by using competent cells (see, e.g., Young and Spizizen, 1961, Journal of Bacteriology 81: 823-829, or Dubnau and Davidoff-Abelson, 1971, Journal of Molecular Biology 56: 209-221), by electroporation (see, e.g., Shigekawa and Dower, 1988, Biotechniques 6: 742-751), or by conjugation (see, e.g., Koehler and Thorne, 1987, Journal of Bacteriology 169: 5271-5278). Specific transformation protocols are known in the art for various types of host cells (see, e.g., for E. coli protoplast transformation see Hanahan, 1983, J. Mol. Biol. 166: 557-580). Various host cells can be used for expressing the nucleic acid construct described herein. Host cells comprising the genetic constructs described herein can be obtained by one of the methods described herein for introducing the polynucleotides into such host cells. The host cell of the present invention does not naturally express the cutinase. Thus, the host cell is a recombinant host cell; the nucleic acid construct described herein is heterologous for the host cell.

In one embodiment, the host cell is a prokaryotic or a eukaryotic cell. In another embodiment, the host cell is a bacterial cell, an archaeal cell, a fungal cell, a yeast cell or a eukaryotic cell. In another embodiment, the host cell is a non-human host cell.

In one embodiment, the host cell is a bacterial cell. The bacterial host cell may be any gram-positive bacterium or a gram-negative bacterium. Gram-positive bacteria include, but are not limited to, Bacillus, Brevibacterium, Corynebacterium, Streptococcus, Streptomyces, Staphylococcus, Enterococcus, Lactobacillus, Lactococcus, Clostridium, Paenibacillus, Geobacillus, and Oceanobacillus. Gram-negative bacteria include, but are not limited to, Escherichia, Pseudomonas, Salmonella, Campylobacter, Basfia, Ensifer, Sinorhizobium, Helicobacter, Acetobacter, Flavobacterium, Fusobacterium, Gluconobacter. In one embodiment, the host cell is a bacterial cell. In a specific embodiment the host cell is of the genus Escherichia or Bacillus. In a specific embodiment, the bacterial host cell is a Bacillus licheniformis cell.

In the methods of the present invention, the bacterial host cell may be any Bacillus cell. Bacillus cells useful in the practice of the present invention include, but are not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus methylotrophicus, Bacillus cereus, Bacillus paralicheniformis, Bacillus subtilis, and Bacillus thuringiensis cells. In one embodiment, the bacterial host cell is a Bacillus amyloliquefaciens, Bacillus pumilus, Bacillus lentus, Bacillus licheniformis, Bacillus stearothermophilus or Bacillus subtilis cell. In preferred embodiment, the bacterial host cell is a Bacillus licheniformis cell, a Bacillus pumilus, or a Bacillus subtilis cell. Preferably, the bacterial host cell is a Bacillus licheniformis cell.

In the methods of the present invention, the bacterial host cell may be Lactobacillus acidophilus, Lactobacillus plantarum, Lactobacillus gasseri, Lactobacillus bulgaricusk, Lactobacillus reuteri, Escherichia coli, Staphylococcus aureus, Corynebacterium glutamicum, Corynebacterium acetoglutamicum, Corynebacterium acetoacidophilum, Corynebacterium callunae, Corynebacterium ammoniagenes, Corynebacterium thermoaminogenes, Corynebacterium melassecola, Corynebacterium effiziens, Corynebacterium efficiens, Corynebacterium deserti, Brevibacterium flavum, Brevibacterium lactofermentum, Brevibacterium divarecatum, Pseudomonas putida, Pseudomonas syringae, Streptomyces coelicolor, Streptomyces lividans, Streptomyces albus, Streptomyces avermitilis, Gluconobacter oxydans, Gluconobacter morbifer, Gluconobacter thailandicus, Acetobacter aceti, Clostridium acetobutylicum, Clostridium saccharobutylicum, Clostridium beijerinckii, Streptococcus equisimilis, Streptococcus pyogenes, Streptococcus uberis, Streptococcus equi subsp., Zooepidemicus or Basfia succiniciproducens.

In another embodiment, the bacterial host cell may additionally contain modifications, e.g., deletions or disruptions, of other genes that may be detrimental to the production, recovery or application of a polypeptide of interest. In one embodiment, a bacterial host cell is a protease-deficient cell. In another embodiment, the bacterial host cell, e.g., Bacillus cell, comprises a disruption or deletion of extracellular protease genes including but not limited to aprE, mpr, vpr, bpr, and / or epr. In one embodiment, the bacterial host cell does not produce spores. In another embodiment, the bacterial host cell, e.g., Bacillus cell, comprises a disruption or deletion of spollAC, sigE, and / or sigG. In one embodiment, the bacterial host cell, e.g., Bacillus cell, comprises a disruption or deletion of one of the genes involved in the biosynthesis of surfactin, e.g., srfA, srfB, srfC, and / or srfD. See, for example, U.S. Patent No. 5,958,728. In another embodiment, the bacterial host cell comprises a disruption or deletion of one of the genes involved in the biosynthesis of polyglutamic acid. Other genes, including but not limited to the amyE gene, which are detrimental to the production, recovery or application of a polypeptide of interest may also be disrupted or deleted.

In another embodiment, the bacterial host cell is a standard E. coli cloning host cell, including but not limited to DH5alpha (Invitrogen), DH10B, (Invitrogen), Omnimax (Invitrogen), INV110 (Invitrogen), TOP10 (Invitrogen), HB101 (Promega), SURE (Stratagene), XL1-Blue (Stratagen), TG1 (Lucigen), and JM109 (NEB). In another embodiment, the bacterial host cell is a standard Bacillus subtilis cloning host cell, including but not limited to B. subtilis carrying a defective hsd(RI)R-M- locus such as B. subtilis IG-20 (BGSC 1A436) or a defective hsdRM1 mutation such as B. subtilis1012 WT (Mobitec).

Alternative further host cells include but are not limited to: Aspergillus niger, Aspergillus oryzae, Hansenula polymorpha, Thermomyces lanuginosus, fusarium oxysporum, Fusarium heterosporum, Pichia pastoris (also known as Komagataella phaffii), Myceliopthora thermophile (C1), Themothelomyces thermophila, Schizosaccharomyces pombe, Trichoderma, preferably Trichoderma reesei and Saccharomyces, preferably Saccharomyces cerevisiae, or Rhizomucor.

### Methods of making

The cutinases described herein can be produced in an industrial scale and subsequently purified. Industrial production of enzymes is usually done by cultivating a host cell, which expresses the enzyme (also called fermentation). Suitable host cells are described above. A nucleic acid sequence encoding the cutinase described herein can be transformed into the host cell, which is subsequently cultivated under conditions suitable for the host cell to produce the cutinase. In a preferred embodiment, the cutinase is purified from the host cell.

Hence, in yet another embodiment, the present invention is directed to a method of producing a cutinase comprising the steps of
(a) providing a host cell comprising a recombinant nucleic acid construct comprising a polynucleotide encoding the cutinase described herein by introducing the nucleic acid construct comprising the polynucleotide encoding the cutinase as described herein into the host cell;
(b) cultivating the recombinant host cell of step (a) under conditions conductive for the expression of the polynucleotide; and
(c) optionally, recovering the cutinase encoded by the polynucleotide.

Cultivation of the host cell normally takes place in a suitable nutrient medium allowing the recombinant cells to grow and express the desired protein. At the end of fermentation, the fermentation broth is collected and may be further processed, wherein the fermentation broth comprises a liquid fraction and a solid fraction. The enzyme of interest may be further purified from the fermentation broth.

The cutinase described herein may be secreted (into the liquid fraction of the fermentation broth) or may not be secreted from the microbial cells (and therefore is comprised in the cells of the fermentation broth). Depending on this, the cutinase may be recovered from the liquid fraction of the fermentation broth or from cell lysates. Preferably, the cutinase is secreted from the cell into the fermentation broth, preferably by means of a secretion signal peptide added to the terminus of the amino acid sequence of the cutinase. Recovery of the cutinase can be achieved by methods known to those skilled in the art. Suitable methods for recovery of proteins from fermentation broth include but are not limited to collection, centrifugation, filtration, extraction, and precipitation. If the product of interest precipitates or crystallizes in the fermentation broth or binds at least in part to the particulate matter of the fermentation broth, additional treatment steps might be needed to release the protein of interest from the biomass or to solubilize crystals and precipitates of the protein of interest. WO0043502A1, WO2008110498A1, and WO2017097869A1 describe a method for recovering a protein of interest from the fermentation broth, which precipitates and/or crystallizes during fermentation. In case the desired protein is comprised in the cells of the fermentation broth, release of the product of interest from the cells might be needed. Release from the cells can be achieved for instance, but not limited thereto, by cell lysis with techniques well known to the skilled person, e.g., lysozyme treatment, ultrasonic treatment, French press or combinations thereof.

The cutinase may be purified from the fermentation broth by methods known in the art. For example, the cutinase may be isolated from the fermentation broth by conventional procedures including, but not limited to, centrifugation, filtration, extraction, spray-drying, evaporation, or precipitation. The isolated polypeptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF)), differential solubility (e.g., ammonium sulfate precipitation), or extraction (see, e.g., Protein Purification, J.-C. Janson and Lars Ryden, editors, VCH Publishers, New York, 1989). The purified polypeptide may then be concentrated by procedures known in the art including, but not limited to, ultrafiltration and evaporation, in particular, thin film evaporation.

### Compositions

The purified solution of the cutinase described herein may be further processed to form a cutinase containing composition. Hence, also claimed herein is a composition comprising a cutinase described herein and at least one additional component.

Thus, the present invention therefore also refers to a method for making a composition comprising the steps of mixing
a) a cutinase as described herein; and
b) one or more components described herein.

The composition can be a non-complex formulation, e.g., a cutinase formulation, or a complex formulation, e.g., a detergent composition.

### Cutinase formulation

In one embodiment of the present invention, the cutinase is formulated as a cutinase formulation, preferably a concentrated cutinase formulation. The cutinase formulation can be either solid or liquid. Such enzyme formulations can be obtained by using techniques known in the art. For instance, without being limited thereto, solid enzyme formulations can be obtained by extrusion or granulation. Suitable extrusion and granulation techniques are known in the art and are described for instance in WO 94/19444 A1 and WO 97/43482 A1.

Liquid cutinase formulations may comprise amounts of enzyme in the range of 0.1% to 40%, 0.5% to 30%, 1% to 25%, 1% to 10%, or preferably 1-6% by weight, all relative to the total weight of the enzyme formulation.

In one embodiment, the cutinase formulation, in particular the liquid enzyme formulation, comprises in addition one or more additional compounds selected from the group consisting of solvent, salt, pH regulator, preservative, enzyme stabilizer, and thickening agent. Preferably, the cutinase formulation is essentially devoid of surfactants. The solvent may be water and/or an organic solvent. The cutinase formulations of the invention may comprise water in amounts of less than about 60% by weight, less than about 50% by weight, less than about 40% by weight or less than about 30% by weight, all relative to the total weight of the enzyme formulation. The cutinase containing formulations of the invention may comprise an organic solvent in amounts of more than 30%, more than 40%, more than about 50% by weight, more than about 60% by weight, more than about 70% by weight, or more than about 80% by weight but less than 99.9%, all relative to the total weight of the enzyme formulation. The organic solvent may be a water-miscible solvent. The organic solvent may be one or more selected from the group consisting of glycerol, propanediol, polypropylene glycol, and polyethylene glycol.

In one embodiment, the cutinase formulation comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1,3-diol, and formic acid in acid form or as its salt, and 4,4'-dichloro 2-hydroxydiphenylether. The liquid compositions of the invention may comprise a preservative in amounts below 10 ppm, such as in amounts ranging from 2 ppm to 5% by weight relative to the total weight of the liquid composition. Preferably, the cutinase formulation is free from preservatives, meaning that preservatives are comprised in amounts less than 1 ppm, preferably 0 ppm.

Preferably, the cutinase formulation comprises an enzyme stabilizing system. The enzyme stabilizing system can be any stabilizing system which is compatible with the cutinase.

Preferably, the enzyme stabilizing system comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (preferably, C₁-C₃) carboxylic acids or salts thereof (preferably, formic acid, formate (preferably, sodium formate), acetic acid, acetate, or lactate).

Preferably, the liquid cutinase formulation comprises or consists of the cutinase, a solvent, an enzyme stabilizing system, and optionally a preservative and optionally a second enzyme different from the cutinase. Preferably, the cutinase formulation is essentially devoid of surfactants. Thus, the present invention therefore also refers to a method for making a cutinase formulation, preferably a concentrated cutinase formulation, comprising the steps of mixing
a) a cutinase as described herein; and
b) one or more components selected from the group consisting of solvent, enzyme stabilizing system, and preservatives
c) and optionally a second enzyme different from the cutinase as described herein.

### Secondary enzymes

In another embodiment, the composition comprising a cutinase as described herein further comprises one or more second enzymes different from the cutinase described herein. Preferably, the second enzyme is selected from the group consisting of proteases, amylases, second lipases, cellulases, mannanases, hemicellulases, phospholipases, esterases, pectinases, lactases, peroxidases, xylanases, second cutinases, pectate lyases, keratinases, reductases, oxidases, phenoloxidases, lipoxygenases, ligninases, pullulanases, tannases, pentosanases, malanases, beta-glucanases, arabinosidases, hyaluronidases, chondroitinases, laccases, nucleases, DNase, phosphodiesterases, phytases, carbohydrases, galactanases, xanthanases, xyloglucanases, oxidoreductase, perhydrolases, aminopeptidase, asparaginase, carbohydrase, carboxypeptidase, catalase, chitinase, cyclodextrin glycosyltransferase, alpha-galactosidase, beta-galactosidase, glucoamylase, alpha-glucosidase, beta-glucosidase, glycosidase, invertase, ribonuclease, transglutaminase, and dispersins. More preferably, the one or more second enzyme is selected from the group consisting of amylases, lipases, cellulases, mannanases, xylanases, DNases, dispersins, pectinases, pectate lyases, glycosidases and oxidoreductases. Most preferably, the second enzyme is a protease or an amylase, preferably a protease.

The composition of the present invention can comprise one type of enzyme or more than one enzyme of different types, e.g., a cutinase and a protease, or more than one enzyme of the same type, e.g., two or more different proteases, or mixtures thereof, e.g., a cutinase and two different proteases.

### Proteases

Enzymes having proteolytic activity are called "proteases" or "peptidases". Proteases are active proteins exerting "protease activity" or "proteolytic activity". Proteolytic activity is related to the rate of degradation of proteins by a protease or proteolytic enzyme in a defined course of time. The methods for analyzing proteolytic activity are well-known in the literature (see e.g. Gupta et al. (2002), Appl. Microbiol. Biotechnol. 60: 381-395). Proteolytic activity may be determined by using Succinyl-Ala-Ala-Pro-Phe-p-nitroanilide (Suc-AAPF-pNA, short AAPF; see e.g. DelMar et al. (1979), Analytical Biochem 99, 316-320) as substrate. pNA is cleaved from the substrate molecule by proteolytic cleavage, resulting in release of yellow color of free pNA which can be quantified by measuring OD₄₀₅.

Proteases are members of class EC 3.4. Proteases include aminopeptidases (EC 3.4.11), dipeptidases (EC 3.4.13), dipeptidyl-peptidases and tripeptidyl-peptidases (EC 3.4.14), peptidyldipeptidases (EC 3.4.15), serine-type carboxypeptidases (EC 3.4.16), metallocarboxypeptidases (EC 3.4.17), cysteine-type carboxypeptidases (EC 3.4.18), omega peptidases (EC 3.4.19), serine endopeptidases (EC 3.4.21), cysteine endopeptidases (EC 3.4.22), aspartic endopeptidases (EC 3.4.23), metallo-endopeptidases (EC 3.4.24), threonine endopeptidases (EC 3.4.25), or endopeptidases of unknown catalytic mechanism (EC 3.4.99).

In one embodiment, the protease may be selected from metallo-endoproteases (EC 3.4.24). A metalloprotease may for example be a thermolysin from, e.g., family M4 or another metalloprotease such as those from M5, M7 or M8 families. A metalloprotease may especially be derived from *Bacillus amyloliquefaciens* described in WO 07/044993A2, from *Bacillus, Brevibacillus, Thermoactinomyces, Geobacillus, Paenibacillus, Lysinibacillus* or *Streptomyces spp.* described in WO 2014194032, WO 2014194054, and WO 2014194117, from *Kribella alluminosa* described in WO 2015193488, or from *Streptomyces* and *Lysobacter* described in WO 2016075078.

In another embodiment, the protease may be selected from serine proteases (EC 3.4.21). Serine proteases or serine peptidases are characterized by having a serine in the catalytically active site, which forms a covalent adduct with the substrate during the catalytic reaction. A serine protease may be selected from the group consisting of chymotrypsin (e.g., EC 3.4.21.1), elastase (e.g., EC 3.4.21.36, EC 3.4.21.37, or EC 3.4.21.71), granzyme (e.g., EC 3.4.21.78 or EC 3.4.21.79), kallikrein (e.g., EC 3.4.21.34, EC 3.4.21.35, EC 3.4.21.118, or EC 3.4.21.119,) plasmin (e.g., EC 3.4.21.7), trypsin (e.g., EC 3.4.21.4), thrombin (e.g., EC 3.4.21.5), and subtilisin. Subtilisin is also known as subtilopeptidase, e.g., EC 3.4.21.62, the latter hereinafter also being referred to as "subtilisin".

A sub-group of the serine proteases are the trypsin-like or chymotrypsin-like proteases, such as trypsin (e.g., of porcine or bovine origin), the *Fusarium* protease described in WO 89/06270 and the chymotrypsin proteases derived from *Cellumonas* described in WO 05/052161 and WO 05/052146.

An additional sub-group of the serine proteases tentatively designated as subtilases has been proposed by Siezen et al. (1991), Protein Eng. 4:719-737 and Siezen et al. (1997), Protein Science 6:501-523. They are defined by homology analysis of more than 170 amino acid sequences of serine proteases previously referred to as subtilisin-like proteases. For a more detailed description of such subtilases and their amino acid sequences reference is made to Siezen et al. (1997), Protein Science 6:501-523.

For the purpose of the invention, the protease may be selected from the following: subtilisin from *Bacillus amyloliquefaciens* BPN' (described by Vasantha et al. (1984) J. Bacteriol. Volume 159, p. 811-819 and JA Wells et al. (1983) in Nucleic Acids Research, Volume 11, p. 7911-7925); subtilisin from *Bacillus licheniformis* (subtilisin Carlsberg; disclosed in EL Smith et al. (1968) in J. Biol Chem, Volume 243, pp. 2184-2191, and Jacobs et al. (1985) in Nucl. Acids Res, Vol 13, p. 8913-8926); subtilisin PB92 (original sequence of the alkaline protease PB92 is described in EP 283075 A2); subtilisin 147 and/or 309 (Esperase^{®}, Savinase^{®}, respectively) as disclosed in WO 89/06279; subtilisin from *Bacillus lentus* as disclosed in WO 91/02792, such as from *Bacillus lentus* DSM 5483 or the variants of *Bacillus lentus* DSM 5483 as described in WO 95/23221; subtilisin from *Bacillus alcalophilus* (DSM 11233) disclosed in DE 10064983; subtilisin from *Bacillus gibsonii* (DSM 14391) as disclosed in WO 2003/054184; subtilisin from *Bacillus sp.* (DSM 14390) disclosed in WO 2003/056017; subtilisin from *Bacillus sp.* (DSM 14392) disclosed in WO 2003/055974; subtilisin from *Bacillus gibsonii* (DSM 14393) disclosed in WO 2003/054184; subtilisin having SEQ ID NO: 4 as described in WO 2005/063974; subtilisin having SEQ ID NO: 4 as described in WO 2005/103244; subtilisin having SEQ ID NO: 7 as described in WO 2005/103244; and subtilisin having SEQ ID NO: 2 as described in application DE 102005028295.4.

The protease may be subtilisin 309 (which might be called Savinase herein) as disclosed as sequence a) in Table I of WO 89/06279 or a variant which is at least 80% identical thereto and has proteolytic activity.

Further examples of useful proteases in accordance with the present invention comprise the variants described in: WO 92/19729, WO 95/23221, WO 96/34946, WO 98/20115, WO 98/20116, WO 99/11768, WO 01/44452, WO 02/088340, WO 03/006602, WO 2004/03186, WO 2004/041979, WO 2007/006305, WO 2011/036263, WO 2011/036264, and WO 2011/072099. Suitable examples comprise especially protease variants of subtilisin protease derived from SEQ ID NO: 22 as described in EP 1921147 (which is the sequence of mature alkaline protease from *Bacillus lentus* DSM 5483) with amino acid substitutions in one or more of the following positions: 3, 4, 9, 15, 24, 27, 33, 36, 57, 68, 76, 77, 87, 95, 96, 97, 98, 99, 100, 101 , 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and 274 (according to the BPN' numbering), which have proteolytic activity. In one embodiment, such a subtilisin protease is not mutated at positions Asp32, His64 and Ser221 (according to BPN' numbering).

The subtilisin may have SEQ ID NO: 22 as described in EP 1921147 (which may be called BLAP WT herein) or is a variant thereof which is at least 80% identical SEQ ID NO: 22 as described in EP 1921147 and has proteolytic activity. In one embodiment, a subtilisin is at least 80% identical to SEQ ID NO: 22 as described in EP 1921147 and is characterized by having amino acid glutamic acid (E), or aspartic acid (D), or asparagine (N), or glutamine (Q), or alanine (A), or glycine (G), or serine (S) at position 101 (according to BPN' numbering) and proteolytic activity. In one embodiment, subtilisin is at least 80% identical to SEQ ID NO: 22 as described in EP 1921147 and is characterized by having amino acid glutamic acid (E), or aspartic acid (D), at position 101 (according to BPN' numbering) and proteolytic activity. Such a subtilisin variant may comprise an amino acid substitution at position 101, such as R101E or R101D, alone or in combination with one or more substitutions at positions 3, 4, 9, 15, 24, 27, 33, 36, 57, 68, 76, 77, 87, 95, 96, 97, 98, 99, 100, 101 , 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and/or 274 (according to BPN' numbering) and has proteolytic activity. In one embodiment, said protease comprises one or more further substitutions: (a) threonine at position 3 (3T), (b) isoleucine at position 4 (4I), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h).

Suitable proteases also include those, which are variants of the above-described proteases which have proteolytic activity. In one embodiment protease variants include variants with at least 40 to 100% identity to the full-length polypeptide sequence of the parent enzyme as disclosed above. In one embodiment protease variants having proteolytic activity are at least 70%, at least 75%, at least 80%, at least 85%, at least 90%, at least 91%, at least 92%, at least 93%, at least 94%, at least 95%, at least 96%, at least 97%, at least 98% or at least 99% identical to the full-length polypeptide sequence of the parent enzyme as disclosed above.

A suitable subtilisin may be at least 80% identical to SEQ ID NO: 22 as described in EP 1921147 and is characterized by comprising one amino acid (according to (a)-(h)) or combinations according to (i) together with the amino acid 101E, 101D, 101N, 101Q, 101A, 101G, or 101S (according to BPN' numbering) and has proteolytic activity.

In one embodiment, a subtilisin is at least 80% identical to SEQ ID NO: 22 as described in EP 1921147 and is characterized by comprising the mutation (according to BPN' numbering) R101E, or S3T + V4I + V205l, or S3T + V4I + R101E + V205I or S3T + V4I + V199M + V205I + L217D or R101E + S156D + L262E and has proteolytic activity.

A subtilisin may have an amino acid sequence being at least 80% identical to SEQ ID NO: 22 as described in EP 1921147 and being further characterized by comprising R101E, and one or more substitutions selected from the group consisting of S156D, L262E, Q137H, S3T, R45E,D,Q, P55N, T58W,Y,L, Q59D,M,N,T, G61 D,R, S87E, G97S, A98D,E,R, S106A,W, N117E, H120V,D,K,N, S125M, P129D, E136Q, S144W, S161T, S163A,G, Y171 L, A172S, N185Q, V199M, Y209W, M222Q, N238H, V244T, N261T,D and L262N,Q,D (as described in WO 2016/096711 and according to the BPN' numbering) and has proteolytic activity.

The protease may be selected from those commercially available including but not limited to those sold under the trade names Alcalase^{®}, Blaze^{®}, Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect^{®} Prime, Purafect MA^{®}, Purafect Ox^{®}, Purafect OxP^{®}, Puramax^{®}, Properase^{®}, FN2^{®}, FN3^{®}, FN4^{®}, Excellase^{®}, Eraser^{®}, Ultimase^{®}, Opticlean^{®}, Effectenz^{®}, Preferenz^{®} and Optimase^{®} (Danisco/DuPont), Axapem^{™} (Gist-Brocases N.V.), *Bacillus lentus* Alkaline Protease (BLAP; sequence shown in Figure 29 of US 5,352,604) and variants thereof and KAP (*Bacillus alkalophilus* subtilisin) from Kao Corporation.

### Lipases

Lipases other than the cutinases as described herein can also be comprised as secondary enzymes in compositions described herein. "Lipases", "lipolytic enzyme", or "lipid esterase" all refer to an enzyme of the EC class 3.1.1 ("carboxylic ester hydrolase"). Lipase means active protein having lipase activity (or lipolytic activity; triacylglycerol lipase, EC 3.1.1.3), cutinase activity (EC 3.1.1.74) other than the cutinases as described herein, sterol esterase activity (EC 3.1.1.13) and/or wax-ester hydrolase activity (EC 3.1.1.50). Lipases include those of bacterial or fungal origin.

In one aspect of the invention, a suitable lipase as secondary enzyme is selected from the following: lipases from *Humicola* (synonym *Thermomyces*), e.g. from *H. lanuginosa* (*T. lanuginosus*) as described in EP 258068, EP 305216, WO 92/05249 and WO 2009/109500 or from *H. insolens* as described in WO 96/13580; lipases derived from *Rhizomucor miehei* as described in WO 92/05249; lipase from strains of *Pseudomonas* (some of these now renamed to *Burkholderia*), e.g. from *P*. *alcaligenes* or *P*. *pseudoalcaligenes* (EP 218272, WO 94/25578, WO 95/30744, WO 95/35381, WO 96/00292), *P*. *cepacia* (EP 331376), *P. stutzeri* (GB 1372034), *P*. *fluorescens, Pseudomonas sp.* strain SD705 (WO 95/06720 and WO 96/27002), *P*. *wisconsinensis* (WO 96/12012), *Pseudomonas mendocina* (WO 95/14783), *P. glumae* (WO 95/35381, WO 96/00292); lipase from *Streptomyces griseus* (WO 2011/150157) and *S*. *pristinaespiralis* (WO 2012/137147), GDSL-type *Streptomyces* lipases (WO 2010/065455); lipase from *Thermobifida fusca* as disclosed in WO 2011/084412; lipase from *Geobacillus stearothermophilus* as disclosed in WO 2011/084417; *Bacillus* lipases, e.g. as disclosed in WO 00/60063, lipases from *B. subtilis* as disclosed in Dartois et al. (1992), Biochemica et Biophysica Acta, 1131, 253-360 or WO 2011/084599, *B. stearothermophilus* (JP S64-074992) or *B. pumilus* (WO 91/16422); lipase from *Candida antarctica* as disclosed in WO 94/01541; cutinase from *Pseudomonas mendocina* (US 5389536, WO 88/09367); cutinase from *Magnaporthe grisea* (WO 2010/107560); cutinase from *Fusarum solani pisi* as disclosed in WO 90/09446, WO 00/34450 and WO 01/92502; and cutinase from *Humicola lanuginosa* as disclosed in WO 00/34450 and WO 01/92502.

Such suitable lipase variants are e.g. those which are developed by methods as disclosed in WO 95/22615, WO 97/04079, WO 97/07202, WO 00/60063, WO 2007/087508, EP 407225 and EP 260105.

Commercially available lipase enzymes include but are not limited to those sold under the trade names Lipolase^{™}, Lipex^{™}, Lipolex^{™} and Lipoclean^{™} (Novozymes A/S), Lumafast (originally from Genencor), Preferenz L (DuPont), and Lipomax (Gist-Brocades/ now DSM).

In one embodiment, lipase is selected from fungal triacylglycerol lipase (EC class 3.1.1.3). Fungal triacylglycerol lipase may be selected from lipases of *Thermomyces lanuginosus.* In one embodiment, the *Thermomyces lanuginosa* lipase is selected from triacylglycerol lipase according to amino acids 1-269 of SEQ ID NO: 2 of US5869438 and variants thereof having lipolytic activity.

*Thermomyces lanuginosa* lipase may be selected from variants having lipolytic activity which are at least 90%, at least 91%, at least 92%, at least 93%, at least 94%, at least 95%, at least 96%, at least 97%, at least 98%, at least 99%, or 100% identical when compared to the full-length polypeptide sequence of amino acids 1-269 of SEQ ID NO: 2 of US5869438. *Thermomyces lanuginosa* lipase may be selected from variants having lipolytic activity comprising conservative mutations only, which do not pertain the functional domain of amino acids 1-269 of SEQ ID NO: 2 of US5869438. Lipase variants of this embodiment having lipolytic activity may be at least 95%, at least 96%, at least 97%, at least 98%, or at least 99% similar when compared to the full-length polypeptide sequence of amino acids 1-269 of SEQ ID NO: 2 of US5869438.

*Thermomyces lanuginosa* lipase may be selected from variants having lipolytic activity comprising the following amino acid substitutions when compared to amino acids 1-269 of SEQ ID NO: 2 of US5869438: T231R and N233R. Said lipase variants may further comprise one or more of the following amino acid exchanges when compared to amino acids 1-269 of SEQ ID NO: 2 of US5869438: Q4V, V60S, A150G, L227G, P256K.

*Thermomyces lanuginosa* lipase may be selected from variants having lipolytic activity comprising the amino acid substitutions T231R, N233R, Q4V, V60S, A150G, L227G, P256K within the polypeptide sequence of amino acids 1-269 of SEQ ID NO: 2 of US5869438 and are at least 95%, at least 96%, or at least 97% similar when compared to the full-length polypeptide sequence of amino acids 1-269 of SEQ ID NO: 2 of US5869438.

*Thermomyces lanuginosa* lipase may be selected from variants having lipolytic activity comprising the amino acid substitutions T231R and N233R within amino acids 1-269 of SEQ ID NO: 2 of US5869438 and are at least 95%, at least 96%, at least 97%, at least 98%, or at least 99% similar when compared to the full-length polypeptide sequence of amino acids 1-269 of SEQ ID NO: 2 of US5869438.

*Thermomyces lanuginosa* lipase may be a variant of amino acids 1-269 of SEQ ID NO: 2 of US5869438 having lipolytic activity, wherein the variant of amino acids 1-269 of SEQ ID NO: 2 of US5869438 is characterized in containing the amino acid substitutions T231R and N233R. *Thermomyces lanuginosa* lipase may be selected from variants having lipolytic activity preferably comprising at least one, preferably more than one, more preferably all of the following substitutions N11K, A18K, G23K, K24A, V77I, D130A, V154I, V187T, T189Q within the polypeptide sequence of amino acids 1-269 of SEQ ID NO: 1 of WO2015/010009 and are at least 95%, at least 96%, or at least 97% similar when compared to the full length polypeptide sequence of amino acids 1-269 of SEQ ID NO: 1 of WO2015/010009.

### Amylases

"Amylases" (alpha and/or beta) include those of bacterial or fungal origin (EC 3.2.1.1 and 3.2.1.2, respectively). Preferably, amylases are selected from the group of alpha-amylases (EC 3.2.1.1).

Amylases according to the invention have "amylolytic activity" or "amylase activity" involving (endo)hydrolysis of glucosidic linkages in polysaccharides.

Amylases maybe from *Bacillus licheniformis* having SEQ ID NO: 2 as described in WO 95/10603 and variants at least 95% identical thereto. Suitable variants are described in WO 95/10603 comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 which have amylolytic activity. Variants are described in WO 94/02597, WO 94/018314, WO 97/043424 and SEQ ID NO: 4 of WO 99/019467.

Amylases further maybe from *B. stearothermophilus* having SEQ ID NO: 6 as disclosed in WO 02/10355 or an amylase with optionally having a C-terminal truncation over the wildtype sequence. Suitable variants of SEQ ID NO: 6 include those comprising a deletion in positions 179 and/or 181 and/or 182 and/or a substitution in position 193.

Amylases further maybe from *Bacillus sp.707* having SEQ ID NO: 6 as disclosed in WO 99/19467 and variants at least 95% thereto. Preferred variants of SEQ NO: 6 are those having a substitution, a deletion or an insertion in one or more of the following positions: R181, G182, H183, G184, N195, I206, E212, E216 and K269.

Amylases further maybe from *Bacillus halmapalus* having SEQ ID NO: 2 or SEQ ID NO: 7 as described in WO 96/23872, also described herein as SP-722. Preferred variants are described in WO 97/3296, WO 99/194671 and WO 2013/001078.

Amylases further may be from *Bacillus sp.* DSM 12649 having SEQ ID NO: 4 as disclosed in WO 00/22103 and variants at least 95% identical thereto.

Amylases may further be from *Bacillus sp.* A 7-7 (DSM 12368) having an amino acid sequence at least 95% identical to SEQ ID NO: 2, in particular over the region of the amino acids 32 to 516 according to SEQ ID NO: 2, as disclosed in WO 02/10356.

Amylases may further be from *Bacillus* strain TS-23 having SEQ ID NO: 2 as disclosed in WO 2009/061380 and variants thereof.

Amylases may further be from *Cytophaga sp.* having SEQ ID NO: 1 as disclosed in WO 2013/184577 and variants at least 95% identical thereto.

Amylases may further be from *Bacillus megaterium* DSM 90 having SEQ ID NO: 1 as disclosed in WO 2010/104675 and variants at least 95% identical thereto.

Amylases may further be from *Bacillus sp.* comprising amino acids 1 to 485 of SEQ ID NO: 2 as described in WO 00/60060 and variants at least 95% identical thereto.

Amylases may further be from *Bacillus amyloliquefaciens* or variants thereof, preferably selected from amylases according to SEQ ID NO: 3 as described in WO 2016/092009. Amylases may have SEQ ID NO: 12 as described in WO 2006/002643 or may be amylase variants thereof comprising the substitutions Y295F and M202LITV within said SEQ ID NO: 12. Amylases may have SEQ ID NO: 6 as described in WO 2011/098531 or may be amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] within said SEQ ID NO: 6.

Amylases may have SEQ ID NO: 1 as described in WO 2013/001078 or may be amylase variants comprising an alteration at two or more (several) positions corresponding to positions G304, W140, W189, D134, E260, F262, W284, W347, W439, W469, G476, and G477 within said SEQ ID NO: 1.

Amylases may have SEQ ID NO: 2 as described in WO 2013/001087 or may be amylase variants comprising a deletion of positions 181+182, or 182+183, or 183+184, within said SEQ ID NO: 2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO: 2.

Amylases may be hybrid alpha-amylases from above mentioned amylases as for example described in WO 2006/066594.

Hybrid amylases may have, according to WO 2014/183920, A and B domains having at least 90% identity to SEQ ID NO: 2 of WO 2014/183920 and a C domain having at least 90% identity to SEQ ID NO: 6 of WO 2014/183920, wherein the hybrid amylase has amylolytic activity; preferably the hybrid alpha-amylase is at least 95% identical to SEQ ID NO: 23 of WO 2014/183920 having amylolytic activity.

Hybrid amylases may have, according to WO 2014/183921, A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 having amylolytic activity.

Hybrid amylases may comprise according to WO 2021/032881 an A and B domain originating from the alpha amylase from *Bacillus sp.* A 7-7 (DSM 12368) and a C domain originating from the alpha-amylase from *Bacillus cereus;* preferably, the A and B domain are at least 75% identical to the amino acid sequence of SEQ ID NO: 42 and the C domain is at least 75% identical to the amino acid sequence of SEQ ID NO: 44 - both sequences as disclosed in WO 2021/032881; more preferably, the hybrid amylase is at least 80% identical to SEQ ID NO: 54 as disclosed in WO 2021/032881.

In one preferred embodiment the amylase is a variant as described in WO 2022/175435.

In one embodiment, at least one amylase is selected from commercially available amylases, which include but are not limited to products sold under the trade names Duramyl^{™}, Termamyl^{™}, Fungamyl^{™}, Stainzyme^{™}, Stainzyme Plus^{™}, Natalase^{™}, Liquozyme X and BAN^{™}, Amplify^{™}, Amplify Prime^{™} (from Novozymes A/S), and Rapidase^{™}, Purastar^{™}, Powerase^{™}, Effectenz^{™} (M100 from DuPont), Preferenz^{™} (S1000, S110 and F1000; from DuPont), Prima-Green^{™} (ALL; DuPont), Optisize^{™} (DuPont).

### Mannanases

"Mannanases" as described herein are enzymes selected from the group of mannan degrading enzymes. The mannan degrading enzyme may be selected from β-mannosidase (EC 3.2.1.25), endo-1,4-β-mannosidase (EC 3.2.1.78), and 1,4-β-mannobiosidase (EC 3.2.1.100). Preferably, the mannan degrading enzyme is selected from the group of endo-1,4-β-mannosidase (EC 3.2.1.78), a group of enzymes which may be called endo-β-1,4-D-mannanase, β-mannanase, or mannanase herein.

The mannanase may be selected from alkaline mannanase of Family 5 or 26 (i.e., GH5 or GH26). The term "alkaline mannanase" is meant to encompass mannanases having an enzymatic activity of at least 40% of its maximum activity at a given pH ranging from 7 to 12, preferably 7.5 to 10.5.

The mannanase may be selected from mannanases originating from Bacillus organisms, such as described in JP-0304706 [beta-mannanase from *Bacillus* sp.], JP-63056289 [alkaline, thermostable beta-mannanase], JP-63036774 [*Bacillus* microorganism FERM P-8856 producing beta-mannanase and beta-mannosidase at an alkaline pH], JP-08051975 [alkaline beta-mannanases from alkalophilic *Bacillus* sp. AM-001], WO 97/11164 [mannanase from *Bacillus amyloliquefaciens*], WO 91/18974 [mannanase active at an extreme pH and temperature], WO 97/11164 [mannanase from *Bacillus amyloliquefaciens*], WO 2014/100018 [endo-(3-mannanase cloned from a *Bacillus circulans* or *Bacillus lentus* strain CMG1240 (Bleman; see US 5,476,775)]. Other suitable mannanases are described in WO 99/064619.

The mannanase may be selected from mannanases originating from *Trichoderma* organisms, such as disclosed in WO 93/24622.

Preferably, the mannanase is a variant as disclosed in any of WO 2021/160816, WO 2021/160818, or WO2021/160820.

Suitable mannanases also include those, which are variants of the above described mannanases, which have mannanase activity. In one embodiment mannanase variants include variants with at least 40 to 100% identity when compared to the full-length polypeptide sequence of the parent enzyme as disclosed above. In one embodiment mannanase variants having mannanase activity are at least 70%, at least 75%, at least 80%, at least 85%, at least 90%, at least 91%, at least 92%, at least 93%, at least 94%, at least 95%, at least 96%, at least 97%, at least 98% or at least 99% similar and/or identical to the full-length polypeptide sequence of the parent enzyme as disclosed above.

The mannanase may be selected from a commercially available mannanase such as Mannaway^{®} (Novozymes A/S) or Preferenz^{®} (M100) (DuPont).

### Cellulases

"Cellulases" are enzymes capable of hydrolyzing cellulose. Cellulases may be selected from cellobiohydrolase (1,4-P-D-glucan cellobiohydrolase, EC 3.2.1.91), endo-ss-1,4-glucanase (EC 3.2.1.4) and ss-glucosidase (EC 3.2.1.21). Endoglucanases of EC class 3.2.1.4 may be named endoglucanase, endo-1,4-ss-D-glucan 4-glucano hydrolase, endo-1,4-beta-glucanase, carboxymethyl cellulase, and beta-1,4-glucanase.

Endoglucanases may be selected from family 5 endoglucanases. Reference is also made to T.-M. Enveri, "Microbial Cellulases" in W.M. Fogarty, Microbial Enzymes and Biotechnology, Applied Science Publishers, p. 183-224 (1983); Methods in Enzymology, (1988) Vol. 160, p. 200-391 (edited by Wood, W.A. and Kellogg, S.T.); Béguin, P., "Molecular Biology of Cellulose Degradation", Annu. Rev. Microbiol. (1990), Vol. 44, pp. 219248; Begun, P. and Aubert, J-P., "The biological degradation of cellulose", FEMS Microbiology Reviews 13 (1994) p.25-58; Henrissat, B., "Cellulases and their interaction with cellulose", Cellulose (1994), Vol. 1, pp. 169-196. Preferably, at least one cellulase comprised in the composition of the invention is selected of the glycosyl hydrolase family 7 (GH7, pfam00840), preferably selected from endoglucanases (EC 3.2.1.4).

Preferably, an alkaline cellulase is used, wherein "alkaline cellulase" is meant to encompass cellulases having enzymatic activity at a given pH ranging from 7 to 12, preferably 7.5 to 10.5.

In one embodiment, the cellulase is selected from cellulases comprising a cellulose binding domain. In one another embodiment, the cellulase comprises a catalytic domain, but no cellulose binding domain.

In one embodiment, the composition of the invention comprises at least one endoglucanases of EC class 3.2.1.4 is originating from
- *Bacillus,* such as *Bacillus sp.* CBS 670.93 and CBS 669.93
- *Melanocarpus,* such as *Melanocarpus albomyces* as disclosed in WO 97/14804
- *Clostridium,* e. g. *Clostridium thermocellum*
- *Humicola,* such as *Humicola insolens* (DSM1800) as disclosed in EP 0495257, EP 0531315, EP 0531372, US 4435307, US 5648263, US 5776757, WO 89/09259, WO 91/17244, WO 94/07998 (sequence displayed in figure 1 43kd human variants thereof), WO 95/24471, WO 96/11262 and WO 98/12307.
- *Fusarium,* such as *Fusarium oxysporum* e. g. strain J79 (DSM2672) as disclosed in EP 0495257, EP 0531315, EP 0531372, US 5648263, US 5776757, WO 89/09259, WO 91/17244, WO 95/24471 and WO 96/11262
- *Thielavia,* such as *Thielavia terrestris* or *Myceliophthora thermophila* strain CBS 11765 as disclosed in EP 0531315, US 5648263, US 5776757, WO 89/09259, WO 91/17244, WO 95/24471, WO 96/11262, WO 96/29397 (SEQ ID NO: 9 and variants thereof), and WO 98/12307.
- *Trichoderma,* such as *Trichoderma reesei, Trichoderma longibrachiatum* or *Trichoderma harzianum* as disclosed in EP 1305432, EP 1240525, WO 92/06165, WO 94/21801, WO 94/26880, WO 95/02043, WO 95/24471 and WO 02/099091.
- *Aspergillus,* such as *Aspergillus aculeatus* as disclosed in WO 93/17244
- *Erwinia,* such as *Erwinia chrysanthermi* as described by M. H. Boyer et. al. in European Journal of Biochemistry, vol. 162, page 311-316 (1987).
- *Acremonium* such as *Acremonium sp., Acremonium persicinum, Acremonium acremonium, Acremonium brachypenium, Acremonium dichromosporum, Acremonium obclavatum, Acremonium pinkertoniae, Acremonium roseogriseum, Acremonium incoloratum,* and *Acremonium furatum* as disclosed in WO 96/11262 and WO 96/29397 (SEQ ID NO: 5 and variants thereof).
- *Cellvibrio* such as *Cellvibrio mixtus* DSM 11683, *Cellvibrio mixtus* DSM 11684, *Cellvibrio mixtus* DSM 11685, *Cellvibrio mixtus* ACM 2601, *Cellvibrio mixtus* DSM 1523, and *Cellvibrio gilvus* DSM 11686, as disclosed in WO 98/08940.
- *Cephalosporium,* such as *Cephalosporium sp.* RYM-202 as disclosed in WO 96/11262.
Suitable cellulases also include those, which are variants of the above described cellulases which have cellulolytic activity. In one embodiment cellulase variants include variants with at least 40 to 100% identity when compared to the full-length polypeptide sequence of the parent enzyme as disclosed above. In one embodiment cellulase variants having cellulolytic activity are at least 70%, at least 75%, at least 80%, at least 85%, at least 90%, at least 91%, at least 92%, at least 93%, at least 94%, at least 95%, at least 96%, at least 97%, at least 98% or at least 99% similar and/or identical to the full-length polypeptide sequence of the parent enzyme as disclosed above.

The cellulase may be a *Humicola insolens* DSM 1800 cellulase complex having endoglucanase, cellobiohydrolase and beta-glucosidase activity.

The cellulase may be a *Humicola insolens* DSM 1800 endoglucanase (EC 3.2.1.4), preferably having the polypeptide sequence according to position 21-435 of SEQ ID NO:2 as disclosed in WO 2018/224544 or variants at least 95% identical thereto.

The cellulase may be a *Humicola insolens* endoglucanase (EC 3.2.1.4) having 43 kD, preferably according to the polypeptide sequence as disclosed in Figure 1a of WO 94/07998 ("43kDhum") or variants thereof which are preferably at least 90% identical thereto, preferably those disclosed in WO 94/07998.

The cellulase may be a *Bacillus sp.* cellulase (EC 3.2.1.4) selected from a polypeptide at least 80% similar and/or identical to the amino acid sequence of position 1 to position 773 of SEQ ID NO: 2 of WO 2004/053039 or a catalytically active fragment thereof. In one embodiment, the cellulase is a mature polypeptide which is at least 95% identical to SEQ ID NO: 1 of WO 2018/224544.

The cellulase may be a *Thielavia terrestris* cellulase (EC 3.2.1.4) having a polypeptide at least 80% similar and/or identical to the amino acid sequence of position 1 to position 299 of SEQ ID NO: 4 of WO 2004/053039 or a catalytically active fragment thereof. In one embodiment, the cellulase is a mature polypeptide which is at least 95% identical to SEQ ID NO: 4 of WO 2018/224544.

The cellulase may be a mature *Sordaria fimicola* cellulase, preferably having a polypeptide sequence according to SEQ ID NO: 5 of WO 2018/224544 or variants at least 95% identical thereto.

At least one cellulase may be selected from Renozyme^{®}, Celluzyme^{®}, Celluclean^{®}, Endolase^{®} and Carezyme^{®} (Novozymes A/S), Clazinase^{™}, and Puradax HA^{™} (Genencor Int. Inc.), and KAC-500(B)^{™} (Kao Corporation).

### Detergent compositions

In one embodiment, the present invention is directed to the use of the cutinases as described herein in a detergent composition. Thus, the present invention is also directed to a detergent composition comprising a cutinase described herein and one or more detergent components. Thus, the present invention also refers to a method for making a detergent composition comprising the steps of mixing
a) a cutinase as described herein; and
b) one or more detergent components described herein.

The one or more detergent component may be selected from the group consisting of additional enzymes different from the cutinase described herein, enzyme stabilizing systems, surfactants, defoamers, builders, polymers, bleaching systems (bleach), rheology modifiers, hydrotropes, softening agents, desiccants, whitening agents, buffers, preservatives, anti-corrosion additives, dyestuff, and fragrances.

Preferably, at least one component of the detergent is selected from the group consisting of surfactants, builders, polymers, preservatives, and second enzymes different to the cutinase. Detergent components may have more than one function in the final application of a detergent composition, therefore any detergent component mentioned in the context of a specific function herein, may also have another function in the final application of a detergent composition. The function of a specific detergent component in the final application of a detergent composition usually depends on its amount within the detergent composition, i.e., the effective amount of a detergent component. Detergent components vary in type and/or amount in a detergent composition depending on the desired application such as washing white textiles, colored textiles, and wool. Further selected component(s) depend on the physical form of a detergent composition (liquid, solid, gel, provided in pouches or as a tablet, etc.). The component(s) chosen e. g. for detergent compositions further depend on regional conventions, which themselves are related to aspects like washing temperatures used, mechanics of laundry machine (vertical vs. horizontal axis machines), water consumption per wash cycle etc. and geographical characteristics like average hardness of water.

In one embodiment, a detergent composition is comprising more than two detergent components, wherein at least one component is effective in stain-removal, at least one component is effective in providing the optimal cleaning conditions, and at least one component is effective in maintaining the physical characteristics of the detergent.

The detergent composition can be a liquid or solid detergent composition or a combination of liquid and solid detergent compositions. The liquid detergent composition is preferably a gel detergent composition. The solid detergent composition can be a soap bar or a powder detergent composition, preferably a powder detergent composition, wherein the powder detergent composition can be pressed to a tablet.

The detergent composition can be a unit dose or multi dose composition. The detergent composition can be in the form of a pouch, including multi-compartment pouches. The detergent composition can be a laundry or dish washing detergent composition, suitable for home care and/or industrial and institutional (I&I) cleaning. Both laundry and dish wash composition can be in the form of a hand wash or automated wash composition. Preferably the dish wash composition is an Automatic Dish Wash (ADW).

Detergent pouches can be of any form, shape, and material, which is suitable for containing the composition, e.g., without allowing the release of the composition from the pouch prior to water contact. The pouch is made from water-soluble film, which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water-soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol co-polymers and, hydroxypropyl methyl cellulose (HPMC). Preferably, the level of polymer in the film for example PVA is at least about 60%. Preferred average molecular weight will typically be about 20,000 to about 150,000. Films can also be blend compositions comprising hydrolytically degradable and water-soluble polymer blends such as polyactide and polyvinyl alcohol (known under the Trade reference M8630 as sold by Chris Craft In. Prod. Of Gary, Ind., US) plus plasticizers like glycerol, ethylene glycerol, Propylene glycol, sorbitol and mixtures thereof. The pouches can comprise a solid laundry detergent composition and/or a liquid detergent composition. The compartment for liquid components can be different in composition from compartments containing solids (see e.g. US 2009/0011970).

Detergent compositions may comprise amounts of enzyme in the range of 0.0002% to 0.09%, preferably 0.0002% to 0.01% by weight, all relative to the total weight of the detergent composition. In one embodiment, the detergent composition has a pH in the range of 5-12, preferably in the range of 6-11, more preferably in a range selected from 6-10, 7-9, and 7.5-8.5. In one embodiment, the composition is a detergent composition, preferably a liquid detergent composition. In one embodiment, the detergent compositions according to the invention comprise one or more surfactant(s). According to its ionic charge, a surfactant is called non-ionic, anionic, cationic, or amphoteric.

The detergent composition of the present invention may comprise one or more surfactants, which may be anionic and/or cationic and/or non-ionic and/or semi-polar and/or zwitterionic, or a mixture thereof. In preferred embodiments, the detergent compositions of the invention comprise at least one surfactant. In a particular embodiment, the detergent composition of the present invention includes a mixture of one or more nonionic surfactants and one or more anionic surfactants. The surfactant(s) is/are typically present at a level of from about 0.1 to 60 wt.-%, such as 1 to 40 wt.-%, 3 to 20 wt.-% or 3 to 10 wt.-%. The surfactant(s) is/are chosen based on the desired cleaning application, and includes any conventional surfactant(s) known in the art.

In one embodiment, the detergent composition of the present invention comprises a reduced amount of surfactant in comparison to a conventional detergent not containing a cutinase as described herein.

Any surfactant known in the art for use in detergents may be utilized. Non-limiting examples of surfactants are disclosed McCutcheon's 2016 Detergents and Emulsifiers, and McCutcheon's 2016 Functional Materials, both North American and International Edition, MC Publishing Co, 2016 edition. Further useful examples are disclosed in earlier editions of the same publications, which are known to those skilled in the art.

The detergent compositions according to the invention may comprise one or more compounds selected from complexing agents (chelating agents (chelants), sequestering agents), precipitating agents, and ion exchange compounds, which may form water-soluble complexes with calcium and magnesium. Such compounds may be called "builders" or "building agents" herein, without meaning to limit such compounds to this function in the final application of a detergent composition.

In one embodiment, the detergent composition of the invention comprises at least one builder selected from non-phosphate-based builders such as sodium gluconate, citrate(s), silicate(s), carbonate(s), phosphonate(s), amino carboxylate(s), polycarboxylate(s), polysulfonate(s), and polyphosphonate(s). In one embodiment, the detergent composition of the invention comprises a strong sequestering builder. Preferably, detergent compositions of the current invention are free from phosphate, meaning essentially free from phosphate-based builders. Herein, "essentially free from phosphate" is to be understood as meaning that the content of phosphate and polyphosphate is in sum in the range of 10 ppm to 1% by weight, determined by gravimetry and referring to the respective inventive detergent composition. In another preferred embodiment, the detergent composition comprises phosphonate, wherein the phosphonate is preferably DTPMP and/or HEDP.

In one embodiment, the detergent compositions of the invention comprise at least one "citrate" selected from the mono- and the dialkali metal salts and in particular the mono- and preferably the trisodium salt of citric acid, ammonium or substituted ammonium salts of citric acid as well as citric acid as such.

Detergent compositions of the invention may comprise one or more silicates. "Silicate(s)" in the context of the present invention include in particular sodium disilicate and sodium metasilicate, aluminosilicates such as sodium aluminosilicates like zeolith A (i.e. Na₁₂(AlO₂)₁₂(SiO₂)₁₂*27H₂O), and sheet silicates, in particular those of the formula alpha-Na₂Si₂O₅, beta-Na₂Si₂O₅, and delta-Na₂Si₂O₅.

Detergent compositions of the invention may comprise one or more carbonates. The term "carbonate(s)" includes alkali metal carbonates and alkali metal hydrogen carbonates, preferred are the sodium salts. Particularly suitable is sodium carbonate (Na₂CO₃).

Detergent compositions of the invention may comprise one or more phosphonates. "Phosphonates" include, but are not limited to 2-phosphinobutane-1,2,4-tricarboxylic acid (PBTC); ethylenediaminetetra(methylenephosphonic acid) (EDTMPA); 1-hydroxyethane-1,1-diphosphonic acid (HEDP), CH₂C(OH)[PO(OH)₂]₂; aminotris(methylenephosphonic acid) (ATMP), N[CHzPO(OH)z]s; aminotris(methylenephosphonate), sodium salt (ATMP), N[CH₂PO(ONa)₂]₃; 2-hydroxyethyliminobis(methylenephosphonic acid), HOCH₂CH₂N[CH₂PO(OH)₂]₂; diethylenetri-aminepenta(methylenephosphonic acid) (DTPMP), (HO)₂POCH₂N[CH₂CH₂N[CH₂PO(OH)₂]₂]₂; diethylenetriaminepenta(methylenephosphonate), sodium salt, C₉H₍₂₈₋ₓ₎N₃NaₓO₁₅P₅ (x=7); hexamethylenediamine(tetramethylenephosphonate), potassium salt, C₁₀H₍₂ₛ₋ₓ₎N₂KₓO₁₂P₄ (x=6); and bis(hexamethylene)triamine(pentamethylenephosphonic acid),
(HO₂)POCH₂N[(CH₂)₂N[CH₂PO(OH)₂]₂]₂. Salts thereof may be suitable, too.

Detergent compositions of the invention may comprise one or more aminocarboxylates. Non-limiting examples of suitable "amino carboxylates" include, but are not limited to: diethanol glycine (DEG), dimethylglycine (DMG), nitrilitriacetic acid (NTA), N-hydroxyethylaminodiacetic acid, ethylenediaminetetraacetic acid (EDTA), N-(2hydroxyethyl)iminodiacetic acid (HEIDA), hydroxyethylenediaminetriacetic acid, N-hydroxyethyl-ethylenediaminetriacetic acid (HEDTA), hydroxyethylenediaminetetraacetic acid, diethylenetriaminepentaacetic acid (DTPA), and methylglycinediacetic acid (MGDA), glutamic acid-diacetic acid (GLDA), iminodisuccinic acid (IDS), hydroxyiminodisuccinic acid, ethylenediaminedisuccinic acid (EDDS), aspartic acid-diacetic acid, and alkali metal salts or ammonium salts thereof. Further suitable are aspartic acid-N-monoacetic acid (ASMA), aspartic acid-N,N-diacetic acid (ASDA), aspartic acid-N-monopropionic acid (ASMP), N-(2-sulfomethyl) aspartic acid (SMAS), N-(2-sulfoethyl) aspartic acid (SEAS), N-(2-sulfomethyl) glutamic acid (SMGL), N-(2-sulfoethyl) glutamic acid (SEGL), N-methyliminodiacetic acid (MIDA), alpha-alanine-N,N-diacetic acid (alpha-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts or ammonium salts thereof. The term "ammonium salts" as used in in this context refers to salts with at least one cation that bears a nitrogen atom that is permanently or temporarily quaternized. Examples of cations that bear at least one nitrogen atom that is permanently quaternized include tetramethylammonium, tetraethylammonium, dimethyldiethyl ammonium, and n-C₁₀-C₂₀-alkyl trimethyl ammonium. Examples of cations that bear at least one nitrogen atom that is temporarily quaternized include protonated amines and ammonia, such as monomethyl ammonium, dimethyl ammonium, trimethyl ammonium, monoethyl ammonium, diethyl ammonium, triethyl ammonium, n-C₁₀-C₂₀-alkyl dimethyl ammonium 2-hydroxyethylammonium, bis(2-hydroxyethyl) ammonium, tris(2-hydroxyethyl)ammonium, N-methyl 2-hydroxyethyl ammonium, N,N-dimethyl-2-hydroxyethylammonium, and especially NH₄⁺.

In one embodiment, detergent compositions of the invention comprise more than one builder. Preferably, inventive detergent compositions contain less than 0.2% by weight of nitrilotriacetic acid (NTA), or 0.01 to 0.1% NTA by weight relative to the total weight of the detergent composition.

In one embodiment, the detergent composition of the invention comprises at least one of aminocarboxylates selected from methylglycine diacetate (MGDA), glutamic acid diacetate (GLDA), and the respective salts thereof, e.g., alkali (such as sodium) salts thereof in amounts in the range of 0.1% to 25.0% by weight, in the range of 1.0% to 18.0% by weight, in the range of 3.0% to 15.0% by weight, in the range of 3.0% to 10.0% by weight, or in the range of 5.0% to 8.0% by weight relative to the total weight of the detergent composition.

The detergent compositions of the invention may comprise one or more hydrotropes. One or more hydrotropes may be selected from organic solvents such as ethanol, isopropanol, ethylene glycol, 1,2-propylene glycol, and further organic solvents known in the art that are water-miscible under normal conditions without limitation. In one embodiment, the detergent composition of the invention comprises 1,2-propylene glycol in a total amount in the range of 5-10% by weight, preferably of about 6% by weight, all relative to the total weight of the detergent composition. Further non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycol ethers, sodium hydroxy naphthoate, sodium hydroxy naphthalene sulfonate, sodium ethyl hexyl sulfate, and combinations thereof.

In one embodiment, the detergent composition comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1,3-diol, and formic acid in acid form or as its salt, and 4,4'-dichloro 2-hydroxydiphenylether. The liquid compositions of the invention may comprise at least one preservative in amounts below 10ppm, such as in amounts ranging from 2 ppm to 5% by weight relative to the total weight of the liquid composition. Preferably, the liquid composition is free from preservatives, meaning that preservatives are comprised in amounts less than 1 ppm.

In one embodiment, the detergent composition comprising a cutinase as described herein further comprises one or more second enzyme different from the cutinase. Preferably, the second enzyme is selected from the group consisting of a protease, amylases, second lipases, cellulases, mannanases, hemicellulases, phospholipases, esterases, pectinases, lactases, peroxidases, xylanases, pectate lyases, keratinases, reductases, oxidases, phenoloxidases, lipoxygenases, ligninases, pullulanases, tannases, pentosanases, malanases, beta-glucanases, arabinosidases, hyaluronidases, chondroitinases, laccases, nucleases, DNase, phosphodiesterases, phytases, carbohydrases, galactanases, xanthanases, xyloglucanases, oxidoreductase, perhydrolases, aminopeptidase, asparaginase, carbohydrase, carboxypeptidase, catalase, chitinase, cyclodextrin glycosyltransferase, alpha-galactosidase, beta-galactosidase, glucoamylase, alpha-glucosidase, beta-glucosidase, invertase, ribonuclease, transglutaminase, and dispersins, and combinations of at least two of the foregoing types. More preferably, the second enzyme is selected from the group consisting of proteases, amylases, lipases, cellulases, mannanases, xylanases, DNases, dispersins, pectinases, pectate lyases, glycosidases, and oxidoreductases, and combinations of at least two of the foregoing types. Most preferably, the second enzyme is an amylase or protease, preferably protease.

Particularly preferred additional enzymes are disclosed elsewhere herein, and that description is incorporated by reference also to this part of the description.

The composition of the present invention can comprise one type of enzyme or more than one enzyme of different types, e.g., an amylase and a protease, or more than one enzyme of the same type, e.g., two or more different proteases, or mixtures thereof, e.g., an amylase and two different proteases.

The detergent compositions may comprise water-soluble sources of calcium and/or magnesium ions. In one embodiment, at the detergent composition comprises an enzyme stabilizing system as described herein.

In one embodiment, the invention relates to a method for providing a detergent composition, preferably a liquid detergent composition, more preferably a liquid laundering detergent composition, comprising the steps of mixing in one or more steps
(a) at least one cutinase according to the invention, preferably wherein the cutinase is provided within a cutinase formulation as described herein; and
(b) at least one detergent component, preferably selected from surfactant, builder, polymer, preservative, and
(c) optionally a second enzyme different to the cutinase as described herein,
   (b) and (c) present in amounts effective for cleaning performance and/or effective in maintaining the physical characteristics of the detergent.

In one embodiment, the present invention is directed to a detergent composition comprising
a) a cutinase as described herein;
b) one or more surfactant, preferably, in a concentration of 0.2-65%, preferably 0.2-40%,
c) one or more builder, preferably, in a concentration of 0.01-25%, and
d) optionally one or more additional compound selected from the group consisting of additional enzyme different from the cutinase under a), defoamer, polymer, bleaching system (bleach), rheology modifier, hydrotrope, softening agent, desiccant, whitening agent, buffer, preservative, anti-corrosion additive, dyestuff and fragrance;
   preferably wherein detergent composition, is a liquid, powder, pouch, or capsule detergent composition.
   Preferably the detergent composition, preferably powder detergent composition, of the present invention comprises in addition to the cutinase as described herein
a) one or more surfactant selected from the group consisting of Alcohol ethoxylate 7EO, Coco fatty acid C12-18, C12-C14- fatty alcohol ether sulfate (2 EO), linear alkyl benzene sulphonic acid, preferably, in a concentration of 0.2-65%,
b) one or more builder selected from the group consisting of HEDP, MGDA, GLDA, and DTPMP, preferably, in a concentration of 0.01-25%, and
c) one or more compound selected from the group consisting of AcetateNa, CitrateNa, Na Silicate, Na Carbonate, Na phospahte, Na hydrogencarbonate, zeolite4A, Na sulfate, Na chloride, optical brightener, and polymers, and optionally Bleach activator and Percarbonate.
   Preferably the detergent composition, preferably liquid detergent composition, of the present invention comprises in addition to the cutinase as described herein
a) one or more surfactant selected from the group consisting of alcohol ethoxylate 7EO, coco fatty acid C12-18, C12C14- fatty alcohol ether sulfate (2 EO), Linear alkyl benzene sulphonic acid, preferably, in a concentration of 0.2-65%,
b) one or more builder selected from the group consisting of HEDP, MGDA, GLDA, and DTPMP, preferably, in a concentration of 0.01-25%, and
c) one or more compound selected from the group consisting of sulphonic acid, 1,2 Propandiol, triethanolamine, monoethanolamine, NaOH, glycerol, ethanol, Na citrate, and polymer.

### Preferred compositions

In one embodiment, the cutinase described herein is included in a composition comprising one or more, preferably all, compounds selected from the group consisting of (all percentages are w/w):
- A formulation comprising a cutinase as described herein, from 0.05% to 0.2%;
- Anionic detersive surfactant (such as alkyl benzene sulphonate, alkyl ethoxylated sulphate and mixtures), from 8% to 15%;
- Non-ionic detersive surfactant (such as alkyl ethoxylated alcohol), from 0.5% to 4%;
- Cationic detersive surfactant (such as quaternary ammonium compounds), from 0 to 4%;
- Other detersive surfactant (such as zwiterionic detersive surfactants, amphoteric surfactants and mixtures thereof), from 0% to 4%;
- Carboxylate polymer (such as co-polymers of maleic acid and acrylic acid), from 1% to 4%;
- Polyethylene glycol polymer (such as a polyethylene glycol polymer comprising poly vinyl acetate side chains), from 0.5% to 4%;
- Polyester soil release polymer (such as Repel-o-tex from and/or Texcare polymers), from 0.1 to 2%;
- Cellulosic polymer (such as carboxymethyl cellulose, methyl cellulose and combinations thereof), from 0.5% to 2%;
- Other polymer (such as amine polymers, dye transfer inhibitor polymers, hexamethylenediamine derivative polymers, and mixtures thereof), from 0% to 4%;
- Zeolite builder and phosphate builder (such as zeolite 4A and/or sodium tripolyphosphate), from 0% to 4 wt%;
- Other builder (such as sodium citrate and/or citric acid), from 0% to 3%;
- Carbonate salt (such as sodium carbonate and/or sodium bicarbonate), from 15% to 30%;
- Silicate salt (such as sodium silicate), from 0% to 10%;
- Filler (such as sodium sulphate and/or bio-fillers), from 10% to 40%;
- Source of available oxygen (such as sodium percarbonate), from 10% to 20%;
- Bleach activator (such as tetraacetylethylene diamine (TAED) and/or nonanoyloxybenzenesulphonate (NOBS), from 2% to 8%;
- Bleach catalyst (such as oxaziridinium-based bleach catalyst and/or transition metal bleach catalyst), from 0% to 0.1%;
- Other bleach (such as reducing bleach and/or pre- formed peracid), from 0% to 10%;
- Chelant (such as ethylenediamine-N'N'-disuccinic acid (EDDS) and/or hydroxyethane diphosphonic acid (HEDP), from 0.2% to 1%;
- Photobleach (such as zinc and/or aluminium sulphonated phthalocyanine), from 0% to 0.1%;
- Hueing agent (such as direct violet 99, acid red 52, acid blue 80, direct violet 9, solvent violet 13 and any combination thereof), from 0% to 1%;
- Brightener (such as brightener 15 and/or brightener 49), from 0.1% to 0.4%;
- Fabric softener (such as montmorillonite clay and/or polydimethylsiloxane (PDMS)), from 0% to 4%;
- Flocculant (such as polyethylene oxide), from 0% to 1%;
- Suds suppressor (such as silicone and/or fatty acid), from 0% to 0.1%;
- Perfume (such as perfume microcapsule, spray-on perfume, starch encapsulated perfume accords, perfume loaded zeolite, and any combination thereof), from 0.1% to 1%; and
- Aesthetics (such as coloured soap rings and/or coloured speckles/noodles), from 0% to 1%; and
- Optionally one or more second enzymes as described herein
   Miscellaneous balance.

In another embodiment, the cutinase described herein is included in a composition comprising one or more, preferably all, compounds selected from the group consisting of (all percentages are w/w):
- A formulation comprising a cutinase as described herein, from 0.05% to 0.2%;
- Carboxyl group-containing polymer (comprising from about 60% to about 70% by mass of an acrylic acid-based monomer (A); and from about 30% to about 40% by mass of a sulfonic acid group-containing monomer (B); and wherein the average molecular weight is from about 23,000 to about 50,000 preferably in the range of from about 25,000 to about 38,000 as described in WO2014032269), from about 0.5 wt% to ab out 1.5 wt%;
- Anionic detersive surfactant (such as alkyl benzene sulphonate, alkyl ethoxylated sulphate and mixtures thereof), from about 8 wt% to about 15 wt%;
- Non-ionic detersive surfactant (such as alkyl ethoxylated alcohol) from about 0.5 wt% to 4 wt%;
- Cationic detersive surfactant (such as quaternary ammonium compounds), from about 0 wt% to about 4 wt%;
- Other detersive surfactant (such as zwiterionic detersive surfactants, amphoteric surfactants and mixtures thereof), from about 0 wt% to 4 wt%;
- Carboxylate polymer (such as co-polymers of maleic acid and acrylic acid) from about 1 wt% to about 4 wt%;
- Polyethylene glycol polymer (such as a polyethylene glycol polymer comprising poly vinyl acetate side chains), from about 0 wt% to about 4 wt%;
- Polyester soil release polymer (such as Repel-O- Tex(R) and/or Texcare(R) polymers), from about 0.1 wt% to about 2 wt%;
- Cellulosic polymer (such as carboxymethyl cellulose, methyl cellulose and combinations thereof) from about 0.5 wt% to about 2 wt%;
- Other polymer (such as amine polymers, dye transfer inhibitor polymers, hexamethylenediamine derivative polymers, and mixtures thereof), from about 0 wt% to about 4 wt%;
- Zeolite builder and phosphate builder (such as zeolite 4A and/or sodium tripolyphosphate), from about 0 wt% to about 4 wt%;
- Other builder (such as sodium citrate and/or citric acid), from about 0 wt% to about 3 wt%;
- Carbonate salt (such as sodium carbonate and/or sodium bicarbonate), from about 15 wt% to about 30 wt%;
- Silicate salt (such as sodium silicate), from about 0 wt% to about 10 wt%;
- Filler (such as sodium sulphate and/or bio-fillers), from about 10 wt% to about 40 wt%;
- Source of available oxygen (such as sodium percarbonate), from about 10 wt% to about 20 wt%;
- Bleach activator (such as tetraacetylethylene diamine (TAED) and/or nonanoyloxybenzenesulphonate (NOBS), from about 2 wt% to about 8 wt%;
- Bleach catalyst (such as oxaziridinium-based bleach catalyst and/or transition metal bleach catalyst), from about 0 wt% to about 0. 1 wt%;
- Other bleach (such as reducing bleach and/or pre-formed peracid), from about 0 wt% to about 10 wt%;
- Chelant (such as ethylenediamine-N'N'-disuccinic acid (EDDS) and/or hydroxyethane diphosphonic acid (HEDP), from about 0.2 wt% to about 1 wt%;
- Photobleach (such as zinc and/or aluminium sulphonated phthalocyanine), from about 0 wt% to about 0. 1 wt%;
- Hueing agent (such as direct violet 99, acid red 52, acid blue 80, direct violet 9, solvent violet 13 and any combination thereof), from about 0 wt% to about 0.5 wt%;
- Brightener (such as brightener 15 and/or brightener 49), from about 0.1 wt% to about 0.4 wt%;
- Fabric softener (such as montmorillonite clay and/or polydimethylsiloxane (PDMS)), from 0 wt% to 15 wt%;
- Flocculant (such as polyethylene oxide), from 0 wt% to 1 wt%;
- Suds suppressor (such as silicone and/or fatty acid), from 0 wt% to 0.1 wt%;
- Perfume (such as perfume microcapsule, spray-on perfume, starch encapsulated perfume accords, perfume loaded zeolite, and any combination thereof), from 0.1 wt% to 1 wt%; and
- Aesthetics (such as colored soap rings and/or colored speckles/noodles), from 0 wt% to 1wt%; and
- Optionally one or more second enzyme as described herein.
- Miscellaneous balance.

The cutinase as described herein can be comprised in one of the following detergent compositions.

| | | wt% in composition |
|---|---|---|
| Maranil DBS/LC | linear alkylbenzene sulfonate, anionic surfactant | 5.5 |
| Edenor coco fatty acid | C₁₂-C₁₈ coco fatty acid | 2.4 |
| Lutensol AO7 | alkyl polyethyleneglycol ether, non-ionic surfactant | 5.5 |
| Texapon N70 | sodium laureth sulfate 2EO, anionic surfactant | 5.5 |
| 1,2 propylene glycol | | 6.0 |
| C₂H₅OH | | 2.0 |
| KOH | | 2.2 |
| Citrate | | 3.0 |
| A formulation comprising a cutinase as described herein and optionally one or more enzymes, preferably a protease | | 0.05 - 0.2% |
| pH | | 8-8.5 |
| Water | | up to 100% |

| | % wt in composition |
|---|---|
| Linear C10C13-alkyl benzene sulfonic acid | 10.0 |
| C12C14- fatty alcohol ether sulfate (2 EO) | 10.0 |
| C12C14-fatty alcohol ethoxylate (7EO) | 20.0 |
| C12C18 coco fatty acid | 5.0 |
| Na-Citrat | 1.5 |
| NaOH 50% | |
| Propylene glycol | 10.0 |
| A formulation comprising a cutinase as described herein and optionally one or more enzymes, preferably a protease | 0.05 - 0.2% |
| water | Ad 100% |
| | |

| | % wt in composition |
|---|---|
| Linear C10C13-alkyl benzene sulfonic acid | 10.0 |
| C12C14- fatty alcohol ether sulfate (2 EO) | 10.0 |
| C12C14-fatty alcohol ethoxylate (7EO) | 20.0 |
| C12C18 coco fatty acid | 5.0 |
| MGDA | 1.5 |
| NaOH 50% | |
| Propylene glycol | 10.0 |
| A formulation comprising a cutinase as described herein and optionally one or more enzymes, preferably a protease | 0.05 - 0.2% |
| Water | Ad 100% |

| | % wt in composition |
|---|---|
| Linear C10C13-alkyl benzene sulfonic acid | 10.0 |
| C12C14- fatty alcohol ether sulfate (2 EO) | 10.0 |
| C12C14-fatty alcohol ethoxylate (7EO) | 20.0 |
| C12C18 coco fatty acid | 5.0 |
| GLDA | 1.5 |
| NaOH 50% | |
| Propylene glycol | 10.0 |
| A formulation comprising a cutinase as described herein and optionally one or more enzymes, preferably a protease | 0.05 - 0.2% |
| Water | Ad 100% |

| | % wt in composition |
|---|---|
| Linear C10C13-alkyl benzene sulfonic acid | 10.0 |
| C12C14- fatty alcohol ether sulfate (2 EO) | 10.0 |
| C12C14-fatty alcohol ethoxylate (7EO) | 20.0 |
| C12C18 coco fatty acid | 5.0 |
| Phosphonate (e.g., HEDP or DTPMP) | 1.5 |
| NaOH 50% | |
| Propylene glycol | 10.0 |
| A formulation comprising a cutinase as described herein and optionally one or more enzymes, preferably an amylase | 0.05 - 0.2% |
| Water | Ad 100% |

| **% wt in** composition | | | | | | | | | | | | |
|---|---|---|---|---|---|---|---|---|---|---|---|---|
| Alcohol ethoxylate 7EO | 0.6 | 0 | 1 | 0 | 0 | 5.2 | 1.2 | 5 | 4 | 0.5 | 0.5 | 0 |
| Coco fatty acid C12-18 | 1.2 | 0 | 0 | 0 | 0 | 0 | 0 | 0 | 0 | 0.3 | 0 | 0.6 |
| C12C14- fatty alcohol ether sulfate (2 EO) | 1.5 | 0 | 0 | 0 | 0 | 6 | 0 | 3.9 | 4.4 | 1.6 | 0 | 0 |
| Linear alkyl benzene sulphonic acid | 12.1 | 11.2 | 13.6 | 21.9 | 18.7 | 12.7 | 7.6 | 12.1 | 11.5 | 12.2 | 6.5 | 10.4 |
| Bleach activator | 0 | 0 | 0 | 0 | 0 | 0 | 0.2 | 9.5 | 9.5 | 0.5 | 0.8 | 2.2 |
| Percarbonate | 0 | 0 | 0 | 0 | 0 | 0 | 3.6 | 19.4 | 16.6 | 2.2 | 11.5 | 5.8 |
| AcetateNa | 0 | 0 | 0 | 0.1 | 0 | 0.1 | 0 | 6.7 | 7.1 | 0.3 | 1 | 0.7 |
| CitrateNa | 0 | 0 | 0 | 0 | 0 | 14 | 0 | 1.6 | 8.2 | 0.3 | 0.9 | 1.7 |
| Na Silicate | 27.9 | 5.8 | 6.6 | 2 | 15 | 20.3 | 3.6 | 11.3 | 16.4 | 10.2 | 9.1 | 16.5 |
| Na Carbonate | 17.2 | 35 | 37.3 | 30.1 | 37 | 1 | 21.6 | 8.7 | 1.4 | 8 | 22.9 | 14.8 |
| Na Phospahte | 0 | 0 | 0 | 14 | 0.3 | 0 | 0 | 0 | 0 | 0 | 0 | 0 |
| Na Hydrogencarbonate | 0.7 | 0.9 | 0.5 | 2.7 | 0.4 | 10.5 | 0.2 | 2.8 | 1.6 | 0.8 | 0.5 | 0.5 |
| Zeolite4A | 4.2 | 0.1 | 5.1 | 10.2 | 1.8 | 11.6 | 1.6 | 1.4 | 2.4 | 1.6 | 1.8 | 2.3 |
| HEDP | 0 | 0 | 0 | 0 | 0 | 0.13 | 0 | 0.27 | 0.16 | 0 | 0 | 0.17 |
| MGDA | 0 | 1.1 | 0 | 0 | 0 | 0 | 0 | 0 | 0 | 0 | 0 | 0 |
| Cellulase | 0-0.5 | 0-0.5 | 0-0.5 | 0-0.5 | 0-0.5 | 0-0.5 | 0-0.5 | 0-0.5 | 0-0.5 | 0-0.5 | 0-0.5 | 0-0.5 |
| Lipase | 0-0.4 | 0-0.4 | 0-0.4 | 0-0.4 | 0-0.4 | 0-0.4 | 0-0.4 | 0-0.4 | 0-0.4 | 0-0.4 | 0-0.4 | 0-0.4 |
| Mannanase | 0-0.4 | 0-0.4 | 0-0.4 | 0-0.4 | 0-0.4 | 0-0.4 | 0-0.4 | 0-0.4 | 0-0.4 | 0-0.4 | 0-0.4 | 0-0.4 |
| Protease* | 0-1.5 | 0-1.5 | 0-1.5 | 0-1.5 | 0-1.5 | 0-1.5 | 0-1.5 | 0-1.5 | 0-1.5 | 0-1.5 | 0-1.5 | 0-1.5 |
| Amylase | 0-0.5 | 0-0.5 | 0-0.5 | 0-0.5 | 0-0.5 | 0-0.5 | 0-0.5 | 0-0.5 | 0-0.5 | 0-0.5 | 0-0.5 | 0-0.5 |
| Cutinase** | 0.05-1.5 | 0.05-1.5 | 0.05-1.5 | 0.05-1.5 | 0.05-1.5 | 0.05-1.5 | 0.05-1.5 | 0.05-1.5 | 0.05-1.5 | 0.05-1.5 | 0.05-1.5 | 0.05-1.5 |
| Na Sulfate | 30.8 | 1.3 | 33 | 11 | 22 | 3 | 51 | 4 | 6 | 57 | 38 | 37 |
| Na Chloride | 0.2 | 43 | 0.1 | 0 | 0.1 | 0.1 | 1 | 1 | 0.5 | 1.2 | 0.2 | 1 |
| optical brightener | 0.02 | 0 | | 0.1 | 0.06 | | | 0.29 | 0.1 | 0.23 | 0.13 | 0.19 |
| Polymers | 1 | 0 | 0.2 | 2 | 0.5 | 3 | 2.2 | 9.2 | 2.2 | 0.7 | | 0.4 |

| | | | | | | | | | | | | |
|---|---|---|---|---|---|---|---|---|---|---|---|---|
| *) preferably the composition comprises a protease inhibitor, preferably selected from phenylboronic acid (preferably 4-FPBA) or a peptide aldehyde or a bisulfite adduct or acetal thereof (preferably a tripeptide aldehyde, preferably, Z-GAY or Z-VAL). **) a cutinase formulation as described herein | | | | | | | | | | | | |

| **% wt in** composition | | | | | | | | | | | | | | |
|---|---|---|---|---|---|---|---|---|---|---|---|---|---|---|
| alcohol ethoxylat 7EO | 5.40 | 10.80 | 12.40 | 7.30 | 1.60 | 7.60 | 3.80 | 0.30 | 13.30 | 8.00 | 5.70 | 20.00 | 9.20 | 29.00 |
| Coco fatty acid C12-18 | 2.40 | 3.10 | 3.20 | 3.20 | 3.50 | 6.40 | 2.80 | 3.00 | 1.70 | 1.80 | 2.50 | 5.00 | 8.60 | 10.40 |
| C12C14- fatty alcohol ether sulfate (2 EO) | 5.40 | 8.80 | 7.10 | 7.10 | 5.40 | 14.00 | 2.80 | 4.50 | 3.90 | 4.10 | | 10.00 | 22.20 | |
| Linear alkyl benzene sulphonic acid | 5.50 | 0.00 | 14.50 | 15.50 | 10.70 | 0.00 | 6.30 | 5.43 | 11.45 | 5.90 | 10.10 | 10.00 | 28.00 | 27.00 |
| 1,2 Propanediol | 6.00 | 3.50 | 8.70 | 8.70 | 1.10 | 7.80 | 0.50 | | 2.50 | 0.40 | 6.00 | 10.00 | 7.00 | 7.00 |
| Triethanolamine | | | | | | | | | | 8.10 | | | | |
| Monoethanolamine | | | 4.00 | 4.30 | 0.30 | | 0.40 | 1.80 | | | | | 8.00 | 7.00 |
| NaOH | 2.20 | 1.10 | | | | 1.00 | | | 2.20 | | 3.30 | 1.50 | | |
| Glycerol | | 0.80 | 3.00 | 2.80 | | | | 0.60 | 0.20 | 1.90 | | | 7.00 | 10.00 |
| Ethanol | 2.00 | | | | 0.38 | 0.39 | | | 1.84 | | | | | |
| Na citrate | 3.00 | 2.80 | 3.40 | 2.10 | 7.40 | 5.40 | 4.60 | 3.30 | 3.30 | 1.40 | | 1.50 | | |
| Polymer | 0-5 | 0-5 | 0-5 | 0-5 | 0-5 | 0-5 | 0-5 | 0-5 | 0-5 | 0-5 | 0-5 | 0-5 | 0-5 | 0-5 |
| Cutinase** | 0.05-1 | 0.05-1 | 0.05-1 | 0.05-1 | 0.05-1 | 0.05-1 | 0.05-1 | 0.05-1 | 0.05-1 | 0.05-1 | 0.05-1 | 0.05-3 | 0.05-3 | 0.05-3 |
| Protease * | 0-1 | 0-1 | 0-1 | 0-1 | 0-1 | 0-1 | 0-1 | 0-1 | 0-1 | 0-1 | 0-1 | 0-3 | 0-3 | 0-3 |
| Amylase | 0-0.5 | 0-0.5 | 0-0.5 | 0-0.5 | 0-0.5 | 0-0.5 | 0-0.5 | 0-0.5 | 0-0.5 | 0-0.5 | 0-0.5 | 0-0.5 | 0-0.5 | 0-0.5 |
| Cellulase | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 |
| Lipase | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 |
| Mannanase | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 | 0-0.2 |
| Pectate Lyase | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 | 0-0.3 |
| water | to 100 | to 100 | to 100 | to 100 | to 100 | to 100 | to 100 | to 100 | to 100 | to 100 | to 100 | to 100 | to 100 | to 100 |

| | | | | | | | | | | | | | | |
|---|---|---|---|---|---|---|---|---|---|---|---|---|---|---|
| *) preferably the composition comprises a protease inhibitor, preferably selected from phenylboronic acid (preferably 4-FPBA) or a peptide aldehyde or a bisulfite adduct or acetal thereof (preferably a tripeptide aldehyde, preferably, Z-GAY or Z-VAL). **) a cutinase formulation as described herein | | | | | | | | | | | | | | |

### Methods of use

The present invention is also directed to the use of a cutinase as described herein in a cleaning process such as for laundry or hard surface cleaning, preferably for home care or I&I cleaning. Thus, the present invention also refers to the use of a cutinase as described herein for providing a detergent composition with improved wash performance, preferably on cutinase sensitive stains, such as plant-based stains and/or fatty stains, preferably fatty stains, preferably sebum stains.

Thus, the present invention therefore also refers to a method for cleaning, preferably laundry or hard surface cleaning, comprising the step of contacting a subject, preferably a textile or a hard surface, with a composition comprising a cutinase as described herein, preferably wherein the composition comprises at least one additional detergent component, preferably a surfactant and/or a builder. Preferably, the hard surfaces are selected from floors, furnishing, walls, sanitary ceramics, glass, metallic surfaces including medical devices, cutlery, and dishes. Preferably, the method for laundry cleaning, comprises the step of contacting a textile with a composition comprising a cutinase as described herein, preferably wherein the composition comprises at least one additional detergent component, preferably a surfactant and/or a builder. Preferably, the method for hard surface cleaning, comprises the step of contacting a medical device, cutlery, or dish with a composition comprising a cutinase as described herein, preferably wherein the composition comprises at least one additional detergent component, preferably a surfactant and/or a builder. A particular preferred form of hard surface cleaning is dishwashing, preferably manual dish washing (MDW) or automatic dishwashing (ADW), most preferably automatic dishwashing (ADW).

Further, the present invention also refers to a method for improving wash performance of a detergent composition, preferably on cutinase-sensitive stains, such as plant-based stains and/or fatty stains, preferably fatty stains, preferably sebum stains, comprising the step of formulating a cutinase as described herein in a detergent composition.

The present invention is also directed to a method of removing plant-based stains and/or fatty stains, preferably fatty stains, more preferably sebum stains, comprising the step of contacting an object comprising a plant-based stain and/or fatty stain, preferably sebum stain, with a composition comprising a polypeptide having cutinase activity comprising an amino acid sequence that is at least 75% identical to SEQ ID NO: 2, at least 80% identical to SEQ ID NO: 4, or at least 84% identical to SEQ ID NO: 6, as described herein.

Thus, the present invention is directed to a method of removing plant-based stains comprising the step of contacting an object comprising a plant-based stain with a composition comprising a polypeptide having cutinase activity comprising an amino acid sequence that is at least 75% identical to SEQ ID NO: 2, at least 80% identical to SEQ ID NO: 4, or at least 84% identical to SEQ ID NO: 6, as described herein.

The present invention is also directed to a method of removing fatty stains, preferably sebum stains, comprising the step of contacting an object comprising a fatty stain, preferably a sebum stain, with a composition comprising a polypeptide having cutinase activity comprising an amino acid sequence that is at least 75% identical to SEQ ID NO: 2, at least 80% identical to SEQ ID NO: 4, or at least 84% identical to SEQ ID NO: 6, as described herein.

The present invention is also directed to the use of a polypeptide having cutinase activity comprising an amino acid sequence that is at least 75% identical to SEQ ID NO: 2, at least 80% identical to SEQ ID NO: 4, or at least 84% identical to SEQ ID NO: 6, as described herein, for the removal of plant-based stains and/or fatty stains, preferably fatty stains, more preferably sebum stains, from an object, preferably a textile or a hard surface.

Additionally, enzymes play an increasing role in animal nutrition, where they are used in feed formulations to improve efficiency while reducing costs by aiding the animal to e. g. digest its feed more efficiently or by detoxification. Therefore, the present invention is also directed to the use of a cutinase as described herein in animal feed. Hence, the present invention is also directed to an animal feed composition comprising a cutinase as described herein.

The present invention also refers to the use of a cutinase as described herein for increasing digestibility of an animal feed.

### Preferred embodiments

Particularly, preferred herein is:
1. A composition comprising a polypeptide having cutinase activity and at least one additional component, wherein the polypeptide having cutinase activity comprises an amino acid sequence that is at least 75% identical to SEQ ID NO: 2, at least 80% identical to SEQ ID NO: 4, or at least 84% identical to SEQ ID NO: 6.
2. A composition comprising a polypeptide having cutinase activity and at least one additional component, wherein the polypeptide having cutinase activity comprises an amino acid sequence that is with increasing preference at least 75%, at least 78%, at least 80%, at least 82%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 2.
3. A composition comprising a polypeptide having cutinase activity and at least one additional component, wherein the polypeptide having cutinase activity comprises an amino acid sequence that is with increasing preference at least 80%, at least 82%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 4.
4. A composition comprising a polypeptide having cutinase activity and at least one additional component, wherein the polypeptide having cutinase activity comprises an amino acid sequence that is with increasing preference at least 84%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 6.
5. A composition comprising a polypeptide having cutinase activity and at least one additional component, wherein the polypeptide having cutinase activity comprises an amino acid sequence that is with increasing preference at least 84%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 2, SEQ ID NO: 4 or SEQ ID NO: 6, more preferably SEQ ID NO: 2 or SEQ ID NO: 6, most preferably SEQ ID NO: 2.
6. A composition according to any of embodiments 1 to 5, wherein the composition is a non-complex formulation, preferably a cutinase formulation, preferably a concentrated cutinase formulation, and wherein the additional component is selected from the group consisting of solvent, salt, pH regulator, preservative, enzyme stabilizer, and thickening agent.
7. A composition according to embodiment 6, wherein the formulation comprises an enzyme stabilizing system, wherein the enzyme stabilizing system preferably comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (preferably, C1-C3) carboxylic acids or salts thereof (preferably, formic acid, formate (preferably, sodium formate), acetic acid, acetate, or lactate.
8. A composition according to embodiments 6 or 7, wherein the composition is liquid and comprises the cutinase in an amount in the range of 0.1% to 40%, 0.5% to 30%, 1% to 25%, 1% to 10%, or preferably 1-6%, all relative to the total weight of the enzyme formulation.
9. A composition according to any of embodiments 1 to 5, wherein the composition is a detergent composition, preferably a laundry detergent composition or a dishwashing detergent composition, and wherein the additional component is at least one detergent component, preferably wherein the detergent composition comprises the cutinase in an amount in the range of 0.0002% to 0.09%, preferably 0.0002% to 0.01% by weight, all relative to the total weight of the detergent composition.
10. The composition according to embodiment 9, wherein the detergent component is selected from the group consisting of surfactants, builders, polymers, bleaching systems, fluorescent whitening agents, suds suppressors, stabilizers, hydrotropes, rheology modifiers, preservatives, and corrosion inhibitors.
11. The composition according to any of embodiments 7 to 10 further comprising at least one second enzyme selected from the group consisting of proteases, amylases, lipases, cellulases, mannanases, xylanases, DNases, dispersins, pectinases, pectate lyases, oxidoreductases, glycosidases, glucoamylases, xanthan lyases, esterases, and additional cutinases, preferably the at least one second enzyme is a protease and/or an amylase, most preferably a protease.
12. The composition of any of embodiments 9 to 11, wherein the composition comprises one or more surfactants and/or one or more builders, preferably a strong sequestering builder.
13. The composition of embodiment 12, wherein the composition comprises a builder wherein the builder is selected from MDGA, GLDA, DTPMP, HEDP, and EDDS, preferably MDGA or EDDS.
14. The composition of any of embodiments 9 to 13, wherein the composition comprises a surfactant, wherein the surfactant is selected from non-ionic surfactant, anionic surfactant, cationic surfactant, amphoteric surfactant, and combinations thereof.
15. The composition of any of embodiments 9 to 14, wherein no anionic surfactant has been added to the detergent composition.
16. The composition of any one of embodiments 9 to 15, wherein the surfactant and/or the builder is bio-degradable and/or bio-based.
17. The composition of any one of embodiments 9 to 16, wherein the detergent composition is liquid or solid.
18. The composition of any one of embodiments 9 to 17, wherein the detergent composition is in the form of a pouch.
19. A method of removing plant-based stains and/or fatty stains, preferably fatty stains, more preferably sebum stains, comprising the step of contacting an object comprising a plant-based stain and/or fatty stain, preferably fatty stain, preferably sebum stain, with a composition according to any of embodiments 1-18, preferably using a detergent composition according to any of embodiments 9-18, preferably the method is a method of removing fatty stains, more preferably sebum stains, comprising the step of contacting an object comprising a fatty stain, preferably sebum stain, with a composition according to any of embodiments 1-18, preferably using a detergent composition according to any of embodiments 9-18.
20. Use of a polypeptide having cutinase activity comprising an amino acid sequence that is at least 75% identical to SEQ ID NO: 2, at least 80% identical to SEQ ID NO: 4, or at least 84% identical to SEQ ID NO: 6 for the removal of plant-based stains and/or fatty stains, preferably fatty stains, more preferably sebum stains.
21. Use of a polypeptide having cutinase activity comprising an amino acid sequence that is with increasing preference at least 75%, at least 78%, at least 80%, at least 82%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 2.
22. Use of a polypeptide having cutinase activity comprising an amino acid sequence that is with increasing preference at least 80%, at least 82%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 4.
23. Use of a polypeptide having cutinase activity comprising an amino acid sequence that is with increasing preference at least 84%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 6.
24. Use of a polypeptide having cutinase activity comprising an amino acid sequence that is with increasing preference at least 84%, at least 85%, at least 88%, at least 90%, at least 91%, at least 92%, at least 93%, at least 94%, at least 95%, at least 96%, at least 97%, at least 98%, at least 99% or 100% identical to SEQ ID NO: 2, SEQ ID NO: 4 or SEQ ID NO: 6, more preferably SEQ ID NO: 2 or SEQ ID NO: 6, most preferably SEQ ID NO: 2.
25. Use of a polypeptide having cutinase activity according to any of embodiments 20 to 24 for the removal of plant-based stains and/or fatty stains, preferably fatty stains, more preferably sebum stains.
26. Use of a polypeptide having cutinase activity according to any of embodiments 20 to 24 in animal feed.
27. An isolated, synthetic, or recombinant polypeptide having cutinase activity comprising an amino acid sequence that is at least 75% identical to SEQ ID NO: 2, at least 80% identical to SEQ ID NO: 4, or at least 84% identical to SEQ ID NO: 6.
28. An isolated, synthetic, or recombinant polypeptide having cutinase activity, wherein the recombinant polypeptide having cutinase activity comprises with increasing preference at least 75%, at least 77%, at least 80%, at least 82%, at least 85%, at least 88%, at least 89%, at least 90%, at least 91%, at least 92%, at least 93%, at least 94%, at least 95%, at least 96%, at least 97%, at least 98%, at least 99% or 100% identity to SEQ ID NO: 2.
29. An isolated, synthetic, or recombinant polypeptide having cutinase activity, wherein the recombinant polypeptide having cutinase activity comprises with increasing preference at least 80%, at least 82%, at least 85%, at least 88%, at least 89%, at least 90%, at least 91%, at least 92%, at least 93%, at least 94%, at least 95%, at least 96%, at least 97%, at least 98%, at least 99% or 100% identity to SEQ ID NO: 4.
30. An isolated, synthetic, or recombinant polypeptide having cutinase activity, wherein the recombinant polypeptide having cutinase activity comprises with increasing preference at least 84%, at least 85%, at least 88%, at least 89%, at least 90%, at least 91%, at least 92%, at least 93%, at least 94%, at least 95%, at least 96%, at least 97%, at least 98%, at least 99% or 100% identity to SEQ ID NO: 6.
31. An isolated, synthetic, or recombinant polypeptide having cutinase activity, wherein the recombinant polypeptide having cutinase activity comprises with increasing preference at least 84%, at least 85%, at least 88%, at least 90%, at least 91%, at least 92%, at least 93%, at least 94%, at least 95%, at least 96%, at least 97%, at least 98%, at least 99% or 100% identity to SEQ ID NO: 2, SEQ ID NO: 4 or SEQ ID NO: 6, more preferably SEQ ID NO: 2 or SEQ ID NO: 6, most preferably SEQ ID NO: 2.
32. A polypeptide according to any of embodiment 27 - 31, wherein the amino acid sequence of the polypeptide having cutinase activity comprises or consists of SEQ ID NO: 2, SEQ ID NO: 4, or SEQ ID NO: 6, more preferably SEQ ID NO: 2 or SEQ ID NO: 6, most preferably SEQ ID NO: 2, or wherein the amino acid sequence of the polypeptide having cutinase activity comprises or consists of SEQ ID NO: 2, SEQ ID NO: 4, or SEQ ID NO: 6, more preferably SEQ ID NO: 2 or SEQ ID NO: 6, most preferably SEQ ID NO: 2, with 1-20 amino acid substitutions, preferably with 1-10 amino acid substitutions or more preferably with 1-5 amino acid substitutions, preferably wherein the amino acid substitutions are conservative amino acid substitutions.
33. An isolated, synthetic, or recombinant polynucleotide encoding the isolated, synthetic, or recombinant polypeptide of any of embodiments 27 to 32, wherein the isolated, synthetic, or recombinant polynucleotide preferably has with increasing preference at least 80%, at least 85%, at least 90%, at least 95%, at least 96%, at least 97%, at least 98%, at least 99%, or 100% sequence identity to SEQ ID NO: 1, SEQ ID NO: 3, or SEQ ID NO: 5, preferably wherein when the polypeptide having cutinase activity has at least 90% sequence identity to SEQ ID NO: 2 the polynucleotide encoding the polypeptide having cutinase activity has at least 70% sequence identity to SEQ ID NO: 1, or wherein when the polypeptide having cutinase activity has at least 90% sequence identity to SEQ ID NO: 4 the polynucleotide encoding the polypeptide having cutinase activity has at least 70% sequence identity to SEQ ID NO: 3, or wherein when the polypeptide having cutinase activity has at least 90% sequence identity to SEQ ID NO: 6 the polynucleotide encoding the polypeptide having cutinase activity has at least 70% sequence identity to SEQ ID NO: 5.
34. A nucleic acid construct comprising the polynucleotide of embodiment 33.
35. An expression vector comprising the polynucleotide of embodiment 33 or the nucleic acid construct of embodiment 34.
36. A host cell comprising the polynucleotide of embodiment 33, the nucleic acid construct of embodiment 34, or the expression vector of embodiment 35.
37. The host cell according to embodiment 36, wherein the host cell is a Bacillus cell, preferably a Bacillus licheniformis cell.
38. A method of making the polypeptide having cutinase activity of embodiments 27-32 comprising providing a polynucleotide encoding the polypeptide having cutinase activity, preferably a polynucleotide according to embodiment 33, transforming the polynucleotide into a host cell, preferably a Bacillus cell, more preferably a Bacillus licheniformis cell, cultivating the host cell to produce the polypeptide having cutinase activity and optionally purifying the polypeptide having cutinase activity.

### Examples

### Example 1: Cutinase expression

104 sequences extracted from public databases were transformed to and expressed in Bacillus. Therefore, plasmid DNA harboring the gene of interest and prepared from *Bacillus subtilis* was used to transform chemically competent *Bacillus licheniformis* by electroporation. The transformed cells were incubated for 2 hours at 37 °C with shaking and then plated on an appropriate selection medium. The plates were incubated for 3 days at 26 °C. Colonies of *Bacillus licheniformis* harboring the gene of interest were used to inoculate 0.6 ml of growth medium in a 96 well plate. The samples were incubated for 16 hours at 30 °C or 37 °C with shaking. The mature seed culture was diluted into an appropriate number of 0.6 ml aliquots in a 96 well plate, and the samples were incubated 48 hours at 30 °C or 37 °C with shaking. The sample was clarified by centrifugation and the supernatant was harvested, aliquoted, and stored at -20 °C. Presence of the gene product of interest is confirmed by LabChip.

18 cultures expressed active cutinases. 5 out of those (SEQ ID NO: 2, 4, 6, 8 and 10) expressed at levels greater than 0.1 mg/ml supernatant when tested in an intermediate expression scale. The intermediate expression follows the same method as described above using multiple wells for each growth that are harvested by centrifugation. The supernatant was harvested, pooled, the cutinase concentration determined by Lab Chip and aliquots were stored at -20 °C. SEQ ID NO: 2 showed particularly high expression of >1 mg/ml supernatant.

### Example 2: Wash performance in terg-o-tometer (TOM)

The wash performance of the 5 well-expressed cutinases (SEQ ID NO: 2, 4, 6, 8 and 10) was tested on different stains in the TOM at 200 rpm and 22 °C for 60 mins. Enzyme was dosed into the detergent composition as depicted in Table 1.

**Table 1: Washing conditions TOM essay**

| | |
|---|---|
| Test equipment | Terg-o-tometer, LP2 Typ, SDL Atlas Inc., USA |
| Washing liquor | 500 ml |
| Washing time and temperature | 60 min at 22 °C |
| Detergent dosage | 3 g /L |
| Fabric/liquor ratio | 1:14 |
| Washing cycles | 1 |
| Water hardness | 2.5 mmol/l Ca²⁺:Mg²⁺:HCO₃⁻ 4:1:8 |
| Test equipment | Terg-o-tometer, LP2 Typ, SDL Atlas Inc., USA |
| Soiled fabric | 5 x 5 cm WFK20D¹⁾ |
| | 5 x 5 cm CS25 spinach¹⁾ |
| | 5 x 5 cm PCS132 sebum bey¹⁾ |
| Laundry compositions | 1) ES1C composition: |
| | Maranil DBS/LC: 5.5 w/w% |
| | Edenor coco fatty acid K12-18: 2.4 w/w% |
| | Texapon N70: 5.4 w/w% |
| | NaOH: 2.2 w/w% |
| | Lutensol AO 7: 5.4 w/w% |
| | 1.2 Propylene glycol: 6 w/w% |
| | Ethanol: 2 w/w% |
| | Sodium citrate: 3 w/w% |
| | Water up to 90% |
| | 2) Tide Hygenic Clean (purchased laundry detergent) |
| Enzyme concentrations | Cutinase: 0.4 ppm in washing liquor |

| | |
|---|---|
| ¹⁾ CFT-CS 06, Producer: Center for Testmaterials BV, NL-3130 AC Vlaardingen | |

The TOM run was followed by three water rinses, drying overnight, scanning of the dried swatches and image analysis by Digimizer to determine RGB values.

Delta RGB values were calculated using the determined RGB values when washing with and without enzyme added to the detergent (ES1C or Tide Hygienic Clean).

**Table 2: Average delta RGB values TOM assay**

| | **WFK20D** | **CS25 spinach** | **PCS132 sebum bey** |
|---|---|---|---|
| *ES1C* | | | |
| SEQ ID NO: 2 | 0,005 | 0,039 | 0,008 |
| SEQ ID NO: 4 | 0,003 | 0,033 | 0,013 |
| SEQ ID NO: 6 | 0,009 | 0,023 | 0,010 |

| *Tide Hygenic Clean* | | | |
|---|---|---|---|
| SEQ ID NO: 2 | 0,009 | 0,011 | 0,018 |
| SEQ ID NO: 4 | 0,002 | 0,015 | 0,016 |
| SEQ ID NO: 6 | 0,004 | 0,001 | 0,037 |

As shown in Table 2, the cutinases of SEQ ID NO: 2, SEQ ID NO: 4, and SEQ ID NO: 6 showed wash performance on the sebum stain WFK20D, a standard sebum stain, as well as on other stains such as CS25 spinach, or PCS132 sebum bey. SEQ ID NO: 8 and SEQ ID NO: 10 did not show any detectable wash performance under the test conditions.

### Example 3: Beaker essay-WFK20D

A smaller scale beaker assay with 50 or 25 mis wash liquor (in 50 ml beaker) was conducted using the stain WFK20D and dosing the cutinases SEQ ID NO: 2 and SEQ ID NO: 6 in different detergent compositions (ES1C, Tide Hygenic Clean, see Table 3).

**Table 3: Washing conditions beaker essay**

| | |
|---|---|
| Test equipment | 50 ml beaker |
| Washing liquor | 50 or 25 ml |
| Washing time and temperature | 45 to 60 min at 22 °C |
| Detergent dosage | ES1C, Tide Hygienic Clean: 3 g /L |
| | |
| Washing cycles | 1 |
| Water hardness | 2.5 mmol/l Ca²⁺:Mg²⁺:HCO₃⁻ 4:1:8 |
| Ballast fabric | none |
| Sum ballast + soiled fabric | |
| Soiled fabric | 2 x 2 cm WFK20D ¹⁾ |
| Laundry composition | 1) ES1C composition: |
| | Maranil DBS/LC: 5.5 w/w% |
| | Edenor coco fatty acid K12-18: 2.4 w/w% |
| | Texapon N70: 5.4 w/w% |
| | NaOH: 2.2 w/w% |
| | Lutensol AO 7: 5.4 w/w% |
| | 1.2 Propylene glycol: 6 w/w% |
| | Ethanol: 2 w/w% |
| | Sodium citrate: 3 w/w% |
| | Water up to 90% |
| | 2) Tide Hygenic Clean (purchased laundry detergent) |
| Enzyme concentrations | Cutinase: 1 ppm in washing liquor |

| | |
|---|---|
| ¹⁾ CFT-CS 06, Producer: Center for Testmaterials BV, NL-3130 AC Vlaardingen | |

The wash liquor was stirred with agitation between 200-250 rpm at 22 °C for 45 min. This was followed by three water rinses, drying overnight, scanning of the dried swatches and image analysis by Digimizer to determine RGB values.

**Table 4: Delta RGB values for beaker essay with stain WFK 20D in 25 ml ES1C detergent**

| | *WFK 20D in ES1C detergent* |
|---|---|
| SEQ ID NO: 2 | 0,053 |
| SEQ ID NO: 6 | 0,023 |

**Table 5: Delta RGB values for beaker essay with stain WFK 20D in 50 ml Tide Hygenic Clean**

| | *WFK 20D in Tide Hygenic Clean* |
|---|---|
| SEQ ID NO: 2 | 0,035 |
| SEQ ID NO: 6 | 0,012 |

As shown in Tables 4 and 5 both cutinases SEQ ID NO: 2 and SEQ ID NO: 6 showed beneficial cleaning effects. The benefit of SEQ ID NO: 2 and SEQ ID NO: 6 in sebum cleaning was clearly demonstrated on the WFK20D stain, a pigment/sebum on polyester/cotton stain. SEQ ID NO: 2 and SEQ ID NO: 6 showed a good improvement in wash performance in both a commercial detergent (Tide Hygenic Clean) as well as in ES1C (3 g/L in hard water). SEQ ID NO: 2 showed better cleaning performance than SEQ ID NO: 6.

## Claims

1. A composition comprising a polypeptide having cutinase activity and at least one additional component, wherein the polypeptide having cutinase activity comprises an amino acid sequence that is at least 75% identical to SEQ ID NO: 2, at least 80% identical to SEQ ID NO: 4, or at least 84% identical to SEQ ID NO: 6.

2. The composition according to claim 1, wherein the composition is a detergent composition, preferably a laundry detergent composition or a dishwashing detergent composition, and wherein the additional component is at least one detergent component.

3. The composition according to claim 2, wherein the detergent component is selected from the group consisting of surfactants, builders, polymers, bleaching systems, fluorescent whitening agents, suds suppressors, stabilizers, hydrotropes, rheology modifiers, preservatives, and corrosion inhibitors.

4. The composition of claims 2 or 3, further comprising at least one second enzyme selected from the group consisting of proteases, amylases, lipases, cellulases, mannanases, xylanases, DNases, dispersins, pectinases, pectate lyases, oxidoreductases, glycosidases, glucoamylases, xanthan lyases, esterases, and additional cutinases.

5. A method of removing plant-based stains and/or fatty stains, preferably fatty stains, more preferably sebum stains, comprising the step of contacting an object comprising a plant-based stain and/or fatty stain, preferably sebum stain, with a composition according to any of claims 1-4.

6. Use of a polypeptide having cutinase activity comprising an amino acid sequence that is at least 75% identical to SEQ ID NO: 2, at least 80% identical to SEQ ID NO: 4, or at least 84% identical to SEQ ID NO: 6 for the removal of plant-based stains and/or fatty stains, preferably fatty stains, more preferably sebum stains, from an object, preferably a textile or a hard surface.

7. An isolated, synthetic, or recombinant polypeptide having cutinase activity comprising an amino acid sequence that is at least 75% identical to SEQ ID NO: 2, at least 80% identical to SEQ ID NO: 4, or at least 84% identical to SEQ ID NO: 6.

8. The isolated, synthetic, or recombinant polypeptide having cutinase activity of claim 7 comprising an amino acid sequence that is at least 80%, at least 85%, at least 90%, or at least 95% identical to SEQ ID NO: 2, SEQ ID NO: 4, or SEQ ID NO: 6, more preferably SEQ ID NO: 2 or SEQ ID NO: 6, most preferably SEQ ID NO: 2.

9. The polypeptide according to claim 7 or claim 8, wherein the amino acid sequence of the polypeptide having cutinase activity comprises or consists of SEQ ID NO: 2, SEQ ID NO: 4, or SEQ ID NO: 6 or wherein the amino acid sequence of the polypeptide having cutinase activity comprises or consists of SEQ ID NO: 2, SEQ ID NO: 4, or SEQ ID NO: 6 with 1-20 amino acid substitutions, preferably with 1-10 amino acid substitutions or more preferably with 1-5 amino acid substitutions, preferably wherein the amino acid substitutions are conservative amino acid substitutions.

10. An isolated, synthetic, or recombinant polynucleotide encoding the polypeptide of any of claims 7-9, wherein the isolated, synthetic, or recombinant polynucleotide preferably has at least 80% sequence identity to SEQ ID NO:1, SEQ ID NO: 3, or SEQ ID NO: 5.

11. A nucleic acid construct comprising the polynucleotide of claim 10.

12. An expression vector comprising the polynucleotide of claim 10 or the nucleic acid construct of claim 11.

13. A host cell comprising the polynucleotide of claim 10, the nucleic acid construct of claim 11, or the expression vector of claim 12.

14. The host cell according to claim 13, wherein the host cell is a *Bacillus* cell.

15. A method of making the polypeptide having cutinase activity of any of claims 7-9, comprising providing a polynucleotide encoding the polypeptide having cutinase activity, transforming the polynucleotide into a host cell, cultivating the host cell to produce the polypeptide having cutinase activity and optionally purifying the polypeptide having cutinase activity.
